# EUROPEAN PATENT APPLICATION

(11) **EP 4 306 518 A1**
(43) Date of publication of application: **17.01.2024**
(21) Application number: 22767560.0
(22) Date of filing: 11.03.2022
(51) Int. Cl.: C07D 403/12, A61K 31/506, A61K 31/5377, A61P 35/00, C07D 403/14

(54) **NOVEL PYRIMIDINE DERIVATIVE SHOWING INHIBITION EFFECT ON GROWTH OF CANCER CELLS**

(30) Priority: 11.03.2021 KR 20210031948
(71) Applicant: Oncobix Co., Ltd., Yongin-si, Gyeonggi-do 16950 (KR)
(72) Inventor: KIM, Sung Eun, Yongin-si, Gyeonggi-do 16950 (KR); LEE, Sun Ho, Yongin-si, Gyeonggi-do 16950 (KR); LEE, Yong Hyup, Yongin-si, Gyeonggi-do 16950 (KR); KONG, Yun Jeong, Yongin-si, Gyeonggi-do 16950 (KR); BAEK, Min Seo, Yongin-si, Gyeonggi-do 16950 (KR); KIM, Min Jung, Yongin-si, Gyeonggi-do 16950 (KR); JEON, Hye Min, Yongin-si, Gyeonggi-do 16950 (KR)
(74) Representative: dompatent von Kreisler Selting Werner - Partnerschaft von Patent- und Rechtsanwälten mbB
(86) International application number: PCT/KR2022/003457
(87) International publication number: WO 2022/191664

(57) **Abstract**

The present invention relates to a novel pyrimidine derivative compound.

## Description

### [Technical Field]

The present invention relates to a novel pyrimidine derivative with excellent inhibition effect on growth of cancer cells, and a method for preparing thereof and a pharmaceutical composition comprising the same.

### [Background Art]

EGFR is a transmembrane protein that is a receptor for epidermal growth factor (EGF) and is a tyrosine kinase that plays a major role in cell regulation by binding to extracellular EGF and transmitting signals into cells. There are four types of EGFR: EGFR1 (erbB-1), HER2/neu (ErbB-2), HER3 (ErbB-3), and HER4 (ErbB-4). It is known that mutations that affect the expression or activity of each EGFR cause abnormal cell division to affect cancer development or cancer progression.

EGFR mutations that cause EGFR over-expression or overactivity (sustained activity) are observed in lung cancer, glioblastoma, and head and neck cancer, and EGFR mutations are observed in more than 30% of all epithelial cancers. In particular, mutations in the epidermal growth factor receptor (EGFR) are observed in more than 50% of non-small cell lung cancers, which are 80-85% of lung cancers in Asians, including Korea.

Various substances have been developed as small molecule therapeutics to inhibit the activity of activated EGFR. In particular, as lung cancer drugs, Gefitinib, Erlotinib, Afatinib, Brigatinib, Icotinib, Osimertinib, Lazertinib etc. have been developed, and as colorectal cancer drugs, Cetuximab, a monoclonal antibody, was developed.

Mutations mainly found in non-small cell lung cancer are Exon 19 deletion, L858R, and Exon 20 insertion. In particular, Exon 20 insertion accounts for 4-10% of all EGFR mutations found in non-small cell lung cancer. For the EGFR 19 deletion or L858R mutation, which accounts for the most frequent EGFR mutations, small molecule EGFR kinase inhibitors such as gefitinib and erlotinib are used as therapeutic agents. However, EGFR exon 20 insertion mutant is not only ineffective against existing tyrosine kinase inhibitors (gefitinib, illotinib, afatinib, etc.), but also has no response to osimertinib and lazertinib, which are targeted agent for EGFR T790M, so targeted therapy is difficult.

Recently, the U.S. Food and Drug Administration (FDA) designated mobocertinib as an innovative therapeutic agent for the treatment of patients with metastatic non-small cell lung cancer with EGFR exon 20 insertion, whose disease has progressed during or after treatment with platinum-based chemotherapy. Currently, lung cancer clinical studies using various EGFR inhibitors as therapeutic agents are being conducted, but there is no drug approved as standard therapy for the treatment of lung cancer with epidermal growth factor receptor exon 20 insertion mutation, so there is an urgent need to develop drugs that can treat it.

Therefore, the present inventors studied EGFR inhibitors targeting the EGFR exon 20 insertion mutant. As a result, the present invention was completed by confirming that the novel pyrimidine derivative compound of the present invention has excellent inhibitory activity against EGFR exon 20 insertion mutant and can be used for the treatment of cancer associated with EGFR mutation.

### [Prior Art Documents]

(Non-Patent Document 1) Annals of Oncology 29(Supplement 1): i3-i9, 2018.
(Non-Patent Document 2) Transl Lung Cancer Res 2019;8 (3) :302-316.

### [Disclosure]

### [Technical Problem]

An example of the present invention is to provide a novel pyrimidine derivative compound that selectively inhibits the growth and drug resistance of cancer cells or cancers having such resistance, induced by EGFR exon 20 insertion mutants, with few side effects.

An example of the present invention is to provide a pharmaceutical composition comprising the novel pyrimidine derivative compound and having antitumor activity by inhibiting the growth of cancer cells.

An example of the present invention is to provide a method for treating a disease caused by an EGFR exon 20 insertion mutant by administering the novel pyrimidine derivative compound to a subject.

An example of the present invention is to provide a use of the pyrimidine derivative compound for the preparation of a pharmaceutical composition for the prevention or treatment of a disease associated with an EGFR exon 20 insertion mutant.

An example of the present invention is to provide a method for preparing the pyrimidine derivative compound.

### [Technical Solution]

In order to achieve the above objects, the present invention provides a novel pyrimidine derivative, the compound represented by Formula I, a solvate, a stereoisomer or a pharmaceutically acceptable salt thereof: 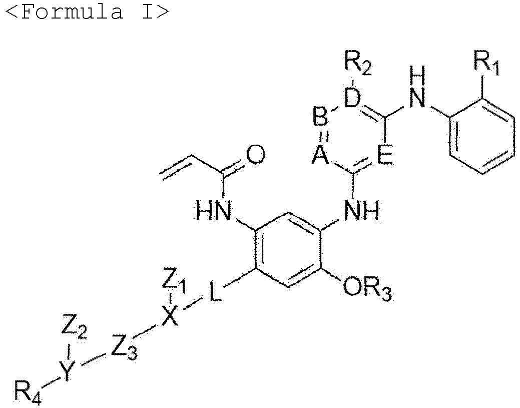 wherein,
A, B, and E are each independently N or C atom,
D is N or C atom,
X is C, N or O atom,
Y is C, N or O atom,
L is a single bond or NR₅, R₅ is H or C1 to C4 alkyl,
Z₁ and Z₂ are each independently C1 to C4 alkyl, or each independently contain carbon and are linked to each other to form a 5- to 8-membered ring with X and Y,
Z₃ is (CH₂)ₙ, or contains carbon and forms a 5- to 8-membered ring with Z₁, Z₂, X and Y, n is an integer from 1 to 3,
R₁ is H, pyrazole, or pyrazole substituted with C1 to C4 alkyl,
R₂ is H, halogen, C1 to C4 alkyl, C1 to C5 alkyl ester (For example, C2 to C5 alkyl ester), CN, or C1 to C4 alkyl substituted with halogen (However, if D is N, R₂ does not exist),
R₃ is H, C1 to C5 linear or branched chain alkyl, C1 to C5 alkyl substituted with halogen, or C3 to C5 cycloalkyl, and
R₄ is not exist, or H, C1 to C4 alkyl, C1 to C4 monoalkyl, or C1 to C4 dialkylamine.

The present invention provides the compound represented by Formula I, a solvate, a stereoisomer or a pharmaceutically acceptable salt thereof used for the treatment of diseases in which exon 20 insertion mutant EGFR is over-expressed.

The present invention provides a pharmaceutical composition for treating a disease over-expressing an EGFR exon 20 insertion mutant gene or protein, comprising the compound represented by Formula I, a solvate, a stereoisomer or a pharmaceutically acceptable salt thereof as an active ingredient and an acceptable carrier.

The present invention provides a method for treating a disease over-expressing an EGFR exon 20 insertion mutant gene or protein comprising administering an effective amount of the compound represented by Formula I, a solvate, a stereoisomer or a pharmaceutically acceptable salt thereof to a subject in need of treatment.

The present invention provides the use of the compound of Formula I, a solvate, a stereoisomer or a pharmaceutically acceptable salt thereof for the preparation of a pharmaceutical composition for preventing or treating a disease over-expressing an EGFR exon 20 insertion mutant gene or protein.

### [Advantageous Effects]

The novel pyrimidine derivative compound can inhibit the growth of cancer cells expressing EGFR exon 20 insertion mutant gene or protein.

Therefore, the novel pyrimidine derivative compound according to the present invention can be used for the treatment of cancer caused by EGFR exon 20 insertion mutant.

### [Best Mode]

Hereinafter, embodiments of the present invention will be described in detail. However, this is presented as an example, and the present invention is not limited thereby, and the present invention is only defined by the scope of the claims described below. In addition, even if it is a configuration essential for carrying out the present invention, a detailed description of a configuration that can be clearly implemented by a person skilled in the art from known technologies will be omitted.

Unless otherwise specified below, the term "compound of the present invention", "compound of Formula I", "compound represented by Formula I", or "pyrimidine derivative compound of Formula I" refers to the compound itself, a solvate, a stereoisomer and salts thereof.

Herein, the term "linear or branched alkyl" means a linear-chain, branched monovalent saturated hydrocarbon group. Unless otherwise defined, the alkyl group typically contains 1 to 5, 1 to 4 or 1 to 3 carbon atoms. Examples of the alkyl group include methyl, ethyl, propyl(e.g. n-propyl and isopropyl), butyl(e.g. n-butyl, isobutyl, and t-butyl), pentyl(e.g. n-pentyl, isopentyl, and neopentyl). Herein, the alkyl group may optionally be partially unsaturated to be an alkenyl or alkynyl below. Further, the alkyl group may be further substituted with other substituents.

Herein, the term "alkoxy" may be linear chain, branched chain or cyclic chain. The number of carbon atoms of the alkoxy group is not particularly limited, but may be, for example, 1 to 5 carbon atoms. Specifically, it may be methoxy, ethoxy, n-propoxy, isopropoxy, n-butoxy, isobutoxy, tert-butoxy, sec-butoxy, n-pentyloxy, neopentyloxy, isopentyloxy, etc., but is not limited thereto.

Herein, the term "haloalkyl" or "alkyl substituted with halogen" refers to an alkyl group with one or more halogen substituents. The haloalkyl includes -CF₃, -C₂F₅, -CHF₂, -CCl₃, - CHCl₂, and -C₂Cl₅. Unless otherwise defined, the haloalkyl group typically contains 1 to 6, 1 to 5, 1 to 4 or 1 to 3 carbon atoms and may be further substituted by other substituents.

Herein, the term "cycloalkyl" refers to non-aromatic carbon rings containing cyclized alkyl, alkenyl and alkynyl groups. The cycloalkyl group may contain monocyclic or polycyclic rings. The polycyclic ring is one having, for example, 2, 3 or 4 fused rings. Unless otherwise defined, the cycloalkyl group typically contains 3 to 5 ring carbon atoms. The cycloalkyl group includes cyclopropyl, cyclobutyl, cyclopentyl, cyclohexyl, cycloheptyl, cyclohexadienyl, cycloheptatrienyl, etc., and may be further substituted by other substituents.

The present invention provides a pyrimidine derivative compound represented by the following Formula I, a solvate, a stereoisomer or a pharmaceutically acceptable salt: 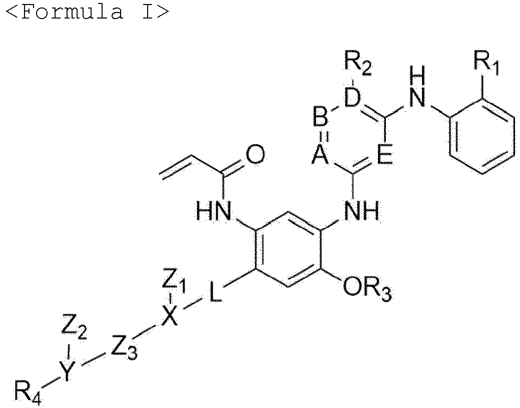 wherein,
A, B, and E are each independently N or CH,
D is N or C,
X is CH, N or O,
Y is CH, N or O,
L is a single bond or NR₅,
R₅ is H or C1 to C4 alkyl,
Z₁ and Z₂ are each independently C1 to C4 alkyl, or each independently contain carbon and are linked to each other to form a 5- to 8-membered ring with X and Y,
Z₃ is (CH₂)ₙ, or contains carbon and forms a 5- to 8-membered ring with Z₁, Z₂, X and Y,
n is an integer from 1 to 3,
R₁ is H, pyrazole, or pyrazole substituted with C1 to C4 alkyl,
R₂ is H, halogen, C1 to C4 alkyl, C1 to C4 alkyl ester, CN, or C1 to C4 alkyl substituted with halogen (However, if D is N, R₂ does not exist),
R₃ is H, C1 to C5 linear or branched chain alkyl, C1 to C5 alkyl substituted with halogen, or C3 to C5 cycloalkyl, and
R₄ is not exist, or H, C1 to C4 alkyl, C1 to C4 monoalkyl, or C1 to C4 dialkylamine.

For example, in Formula I above, the A and D may be N.

For example, in Formula I above, the A and E may be N.

For example, in Formula I above, the Z₁ and Z₂ may each independently contain carbon and may be connected to each other to form a 5- to 8-membered monocyclic, fused bicyclic, or bridged bicyclic ring with X and Y. At this time, in Formula I, the Z₃ may contain carbon atom and form a 5- to 8-membered ring with Z₁, Z₂, X and Y, and may be a heterocyclic ring. The heterocyclic ring may include one or more hetero atoms selected from the group consisting of O, S, N, and P. For example, the heterocyclic ring may include one or more hetero atoms selected from the group consisting of O and N. For example, the heterocyclic ring may include 1 to 2 hetero atoms.

For example, in Formula I, when L is a single bond, X may be N or O.

For example, in Formula I, when L is a single bond, X may be N.

For example, in Formula I, when L is NR₅, X may be CH.

For example, in Formula I, when Y is O, R₄ may not exist.

For example, the compound of Formula I may be any one of the following compounds:
N-(2-((2-(dimethylamino)ethyl)(methyl)amino)-4-methoxy-5-((6-((2-(1-methyl-1H-pyrazol-3-yl)phenyl)amino)pyrimidin-4-yl)amino)phenyl)acrylamide;
N-(5-((6-((2-(1H-pyrazol-1-yl)phenyl)amino)pyrimidin-4-yl)amino)-2-((2-(dimethylamino)ethyl)(methyl)amino)-4-methoxyphenyl)acrylamide;
N-(2-((2-(dimethylamino)ethyl)(methyl)amino)-4-isopropoxy-5-((6-((2-(1-methyl-1H-pyrazol-3-yl)phenyl)amino)pyrimidin-4-yl)amino)phenyl)acrylamide;
N-(4-methoxy-5-((6-((2-(1-methyl-1H-pyrazol-3-yl)phenyl)amino)pyrimidin-4-yl)amino)-2-(4-methylpiperizin-1-yl)phenyl)acrylamide;
N-(4-methoxy-5-((6-((2-(1-methyl-1H-pyrazol-3-yl)phenyl)amino)pyrimidin-4-yl)amino)-2-morpholinophenyl)acrylamide;
(S)-N-(2-(3-(dimethylamino)pyrrolidin-1-yl)-4-methoxy-5-((6-((2-(1-methyl-1H-pyrazol-3-yl)phenyl)amino)pyrimidin-4-yl)amino)phenyl)acrylamide;
(R)-N-(2-(3-(dimethylamino)pyrrolidin-1-yl)-4-methoxy-5-((6-((2-(1-methyl-1H-pyrazol-3-yl)phenyl)amino)pyrimidin-4-yl)amino)phenyl)acrylamide;
(R)-N-(4-methoxy-2-(methyl(1-methylpyrrolidin-3-yl)amino)-5-((6-((2-(1-methyl-1H-pyrazol-3-yl)phenyl)amino)pyrimidin-4-yl)amino)phenyl)acrylamide;
(S)-N-(4-methoxy-2-(methyl(1-methylpyrrolidin-3-yl)amino)-5-((6-((2-(1-methyl-1H-pyrazol-3-yl)phenyl)amino)pyrimidin-4-yl)amino)phenyl)acrylamide;
N-(2-((2-(diamethylamino)ethyl)(methyl)amino)-5-((6-((2-(1-methyl-1H-pyrazol-3-yl)phenyl)amino)pyrimidin-4-yl)amino)-4-(2,2,2-trifluoroethoxy)phenyl)acrylamide;
N-(2-((2-(dimethylamino)ethyl)(methyl)amino)-4-ethoxy-5-((6-((2-(1-methyl-1H-pyrazol-3-yl)phenyl)amino)pyrimidin-4-yl)amino)phenyl)acetamide;
N-(4-cyclopropoxy-2-((2-(dimethylamino)ethyl)(methyl)amino)-5-((6-((2-(1-methyl-1H-pyrazol-3-yl)phenyl)amino)pyrimidin-4-yl)amino)phenyl)acrylamide;
N-(5-((5-cyano-4-((2-(1-methyl-1H-pyrazol-3-yl)phenyl)amino)pyrimidin-2-yl)amino)-2-((2-(dimethylamino)ethyl)(methyl)amino)-4-methoxyphenyl)acrylamide;
N-(5-((4-((2-(1H-pyrazol-1-yl)phenyl)amino)-5-methylpyrimidin-2-yl)amino)-2-((2-(dimethylamino)ethyl)(methyl)amino)-4-methoxyphenyl)acrylamide;
N-(2-((2-(dimethylamino)ethyl)(methyl)amino)-4-methoxy-5-((4-((2-(1-methyl-1H-pyrazol-3-yl)phenyl)amino)-5-(trifluoromethyl)pyrimidin-2-yl)amino)phenyl)acrylamide;
Isopropyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate;
Isopropyl 4-((2-(1H-pyrazol-1-yl)phenyl)amino)-2-((5-acrylamido-4-((2-(dimethylamino)ethyl)(methyl)amino)-2-methoxyphenyl)amino)pyrimidin-5-carboxylate;
methyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate;
Methyl 4-((2-(1H-pyrazol-1-yl)phenyl)amino)-2-((5-acrylamido-4-((2-(dimethylamino)ethyl)(methyl)amino)-2-methoxyphenyl)amino)pyrimidin-5-carboxylate;
Ethyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate;
Ethyl 4-((2-(1H-pyrazol-1-yl)phenyl)amino)-2-((5-acrylamido-4-((2-(dimethylamino)ethyl)(methyl)amino)-2-methoxyphenyl)amino)pyrimidin-5-carboxylate;
Cyclopropyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate;
Isopropyl 2-((5-acrylamido-2-methoxy-4-(4-methylpiperizin-1-yl)phenyl)amino)-4-((2-(1-methyl-1H-pyrazol-3-yl)phenyl)amino)pyrimidin-5-carboxylate;
Isopropyl 2-((5-acrylamido-2-methoxy-4-morpholinophenyl)amino)-4-((2-(1-methyl-1H-pyrazol-3-yl)phenyl)amino)pyrimidin-5-carboxylate;
Isopropyl(S)-2-((5-acrylamido-4-(3-(dimethylamino)pyrrolidin-1-yl)-2-methoxyphenyl)amino)-4-((2-(1-methyl-1H-pyrazol-3-yl)phenyl)amino)pyrimidin-5-carboxylate;
Isopropyl(R)-2-((5-acrylamido-4-(3-(dimethylamino)pyrrolidin-1-yl)-2-methoxyphenyl)amino)-4-((2-(1-methyl-1H-pyrazol-3-yl)phenyl)amino)pyrimidin-5-carboxylate;
Isopropyl(R)-2-((5-acrylamido-2-methoxy-4-(methyl(1-methylpyrrolidin-3-yl)amino)phenyl)amino)-4-((2-(1-methyl-1H-pyrazol-3-yl)phenyl)amino)pyrimidin-5-carboxylate;
Isopropyl(S)-2-((5-acrylamido-2-methoxy-4-(methyl(1-methylpyrrolidin-3-yl)amino)phenyl)amino)-4-((2-(1-methyl-1H-pyrazol-3-yl)phenyl)amino)pyrimidin-5-carboxylate;
N-(5-((5-chloro-4-((2-(1-methyl-1H-pyrazol-3-yl)phenyl)amino)pyrimidin-2-yl)amino)-2-((2-(dimethylamino)ethyl)(methyl)amino)-4-methoxyphenyl)acrylamide;
N-(5-((4-((2-(1H-pyrazol-1-yl)phenyl)amino)-5-chloropyrimidin-2-yl)amino)-2-((2-(dimethylamino)ethyl)(methyl)amino)-4-methoxyphenyl)acrylamide;
N-(5-((5-chloro-4-((2-(1-methyl-1H-pyrazol-3-yl)phenyl)amino)pyrimidin-2-yl)amino)-4-methoxy-2-(4-methylpiperizin-1-yl)phenyl)acrylamide;
N-(5-((5-chloro-4-((2-(1-methyl-1H-pyrazol-3-yl)phenyl)amino)pyrimidin-2-yl)amino)-4-methoxy-2-morpholinophenyl)acrylamide;
(S)-N-(5-((5-chloro-4-((2-(1-methyl-1H-pyrazol-3-yl)phenyl)amino)pyrimidin-2-yl)amino)-2-(3-(dimethylamino)pyrrolidin-1-yl)-4-methoxyphenyl)acrylamide;
(R)-N-(5-((5-chloro-4-((2-(1-methyl-1H-pyrazol-3-yl)phenyl)amino)pyrimidin-2-yl)amino)-2-(3-(dimethylamino)pyrrolidin-1-yl)-4-methoxyphenyl)acrylamide;
(R)-N-(5-((5-chloro-4-((2-(1-methyl-1H-pyrazol-3-yl)phenyl)amino)pyrimidin-2-yl)amino)-4-methoxy-2-(methyl(1-methylpyrrolidin-3-yl)amino)phenyl)acrylamide;
(S)-N-(5-((5-chloro-4-((2-(1-methyl-1H-pyrazol-3-yl)phenyl)amino)pyrimidin-2-yl)amino)-4-methoxy-2-(methyl(1-methylpyrrolidin-3-yl)amino)phenyl)acrylamide,
N-(5-((6-((2-(1H-pyrazol-1-yl)phenyl)amino)pyrimidin-4-yl)amino)-2-((2-(diethylamino)ethyl)(methyl)amino)-4-methoxyphenyl)acrylamide;
N-(5-((6-((2-(1H-pyrazol-1-yl)phenyl)amino)pyrimidin-4-yl)amino)-4-methoxy-2-(tetrahydro-1H-furo[3,4-c]pyrrol-5(3H)-yl)phenyl)acrylamide; and
N-(5-((6-((2-(1H-pyrazol-1-yl)phenyl)amino)pyrimidin-4-yl)amino)-2-(3-oxa-8-azabicyclo[3.2.1]octan-8-yl)-4-methoxyphenyl)acrylamide.

The compounds of Formula I according to one embodiment of the present invention inhibited the growth of EGFR exon 20 insertion mutant lung cancer cell line and induced apoptosis. Specifically, the compounds in the Examples of the present invention have IC₅₀ values of 25 nM or less, 30 nM or less, 35 nM or less, 40 nM or less, 45 nM or less, 50 nM or less, 500 nM or less, or 1000 nM or less.

In addition, the compound of Formula I according to one embodiment of the present invention is more effective than mobocertinib, a control drug previously developed as an anticancer drug for cancer patients with an EGFR exon 20 insertion mutant, and has an activity equivalent to that of poziotinib, which has toxicity problems.

Taken together, the compound of Formula I according to one embodiment of the present invention can be used as a pharmaceutical composition, that is, as an anticancer agent, capable of treating cancer having an EGFR exon 20 insertion mutant.

In addition, the compound of Formula I according to one embodiment of the present invention may inhibit not only EGFR exon 20 insertion but also other EGFR subtypes.

The cancer may be selected from the group consisting of liver cancer, hepatocellular carcinoma, gastrointestinal cancer, stomach cancer, meningioma associated with neurofibromatosis, pancreatic cancer, leukemia, myeloproliferative disease, myelodysplastic disease, dermatofibrosarcoma, breast cancer, lung cancer, thyroid cancer, colorectal cancer, prostate cancer, ovarian cancer , brain tumor, head and neck cancer and glioblastoma. Also, the lung cancer may be non-small cell lung cancer. In addition, the cancer may be a secondary cancer that has metastasized to other organs from the above various types of cancer.

The compound according to one embodiment of the present invention may be in the form of a pharmaceutically acceptable salt thereof. The salt refers to a salt commonly used in the pharmaceutical industry to which the present invention belongs. For example, the salt can be used in the form of a salt induced by at least one acid selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid , maleic acid, benzoic acid, hydroxybenzoic acid, phenylacetic acid, cinnamic acid, salicylic acid, methanesulfonic acid, ethanesulfonic acid, benzenesulfonic acid, toluenesulfonic acid, and the like, and the types of salts meant in the present invention are not limited by the listed salts.

The compound according to one embodiment of the present invention may be in the form of a solvate thereof. The "solvate" means a complex or aggregate formed by one or more solute molecules, i.e., the compound of Formula I or a pharmaceutically acceptable salt thereof, and one or more solvent molecules. The solvate can be a complex or aggregate formed with various solvent molecules, for example water, methanol, ethanol, isopropanol or acetic acid.

The compound according to one embodiment of the present invention may be in the form of a stereoisomer thereof. The stereoisomer includes all stereoisomers such as enantiomers and diastereomers. The compound may be in stereoisomerically pure form or a mixture of one or more stereoisomers, for example a racemic mixture. Isolation of a specific stereoisomer can be performed by one of the conventional methods known in the art. In some embodiments of the compound of the present invention, the anticancer effect of a particular stereoisomer may be greater than that of a racemic mixture. In this case, the dosage can be reduced by using a specific stereoisomer. Therefore, cancer can be efficiently treated by isolating a specific stereoisomer, such as an enantiomer or a diastereoisomer, which has a high killing effect on cancer cells.

Another aspect of the present invention provides a pharmaceutical composition comprising a therapeutically effective amount of the compound of Formula I as defined above, or a pharmaceutically acceptable salt or a solvate or a stereoisomer thereof, and a pharmaceutically acceptable carrier.

In the composition of the present invention, the compound, or the pharmaceutically acceptable salt or solvate or stereoisomer thereof is as described above.

In the composition of the present invention, "Pharmaceutically acceptable carrier" refers to a substance, usually an inert substance, used in combination with an active ingredient to assist in the application of the active ingredient. The carrier includes conventional pharmaceutically acceptable excipients, additives or diluents. The carrier may include, for example, at least one selected from a filler, a binder, a disintergrant, a buffer, a preservatives, an antioxidant, a lubricant, a flavoring agent, a thickener, a coloring agent, an emulsifier, a suspending agent, a stabilizer, a pH adjusting agent, and an isotonic agent.

It may be selected from the group consisting of microcrystalline cellulose, lactose monohydrate, lactose anhydride, lactose, starch, mannitol, carboxymethylcellulose, sorbitol, and combinations thereof, but is not limited thereto. The binder may be selected from the group consisting of hydroxypropyl cellulose, hydroxypropyl methylcellulose, hypromellose, polyvinylacetic acid, povidone, polyvinylpyrrolelidone, copovidone, macrogol, sodium lauryl sulfate, light anhydrous silicic acid, synthetic aluminum silicate, calcium silicate or silicate derivatives such as magnesium metasilicate aluminate, phosphates such as calcium hydrogen phosphate, carbonates such as calcium carbonate, pregelatinized starch, gums such as acacia gum, gelatin, cellulose derivatives such as ethylcellulose, and mixtures thereof, but is not limited thereto.

The disintegrant may be selected from the group consisting of low-substituted hydroxypropyl cellulose, crospovidone, croscarmellose sodium, sodium starch glycolate, F-melt, and combinations thereof, but is not limited thereto. The glidant may be selected from the group consisting of colloidal silicon dioxide, hydrated silicon dioxide, and combinations thereof, but is not limited thereto.

The lubricant may be selected from the group consisting of magnesium stearate, silicon dioxide, talc, light anhydrous silicic acid, sodium stearyl fumarate, and combinations thereof, but is not limited thereto.

The pH adjusting agent may be an acidifying agent such as acetic acid, ascorbic acid, malic acid, succinic acid, tartaric acid, fumaric acid and citric acid and a basifying agent such as precipitated calcium carbonate, aqueous ammonia, meglumine, sodium carbonate, magnesium oxide, magnesium carbonate, sodium citrate and tribasic calcium phosphate.

The antioxidant may be dibutylhydroxytoluene, butylated hydroxyanisole, tocopherol acetate, tocopherol, propyl gallate, sodium hydrogensulfite and sodium pyrosulfite. The solubilizing agent may be polyoxyethylene sorbitan fatty acid esters such as sodium lauryl sulfate and polysorbate, sodium docusate, and poloxamer.

In addition, the formulation of the present invention may be formulated by using pharmaceutically acceptable additives as various additives selected from coloring agents and flavoring agents.

In the present invention, the scope of the additives is not limited to the use of the additives above, and formulation can be carried out by selecting the above-described additives and containing a dose in the usual range.

The pharmaceutical composition according to one embodiment of the present invention is formulated and used in the form of oral formulations such as powders, granules, tablets, capsules, suspensions, emulsions, syrups and aerosols, external preparations, suppositories or sterile injection solutions according to conventional methods.

The composition according to one embodiment of the present invention may be administered orally or parenterally, including intravenous, intraperitoneal, subcutaneous, rectal and topical administration.

Another aspect of the invention provides a method of treating a disease in a subject comprising administering to the subject a therapeutically effective amount of the compound of Formula I, or a pharmaceutically acceptable salt or a solvate or a stereoisomer thereof.

In the above method, a person skilled in the art can appropriately select an administration route according to the patient's condition. The administration may be oral or parenteral. The parenteral administration includes intravenous, intraperitoneal, subcutaneous, rectal and topical administration.

In the above method, the dosage may be variously changed according to various factors such as the condition of the patient, the route of administration, the judgment of the attending physician, and the like, as described above. Effective doses can be estimated from dose-response curves obtained in vitro or in animal model tests. The ratio and concentration of the compound of the present invention present in the composition to be administered may be determined according to chemical characteristics, route of administration, therapeutic dose, and the like. The dosage can be administered to an individual in an effective amount of about 1 g/kg/day, or about 0.1 mg/kg/day to about 500 mg/kg/day. The dose may be changed according to the age, weight, sensitivity, or symptoms of the subject.

Furthermore, the pharmaceutical composition comprising the compound of Formula I of the present invention or a solvate, a stereoisomer or a pharmaceutically acceptable salt thereof as an active ingredient can be used in a method for preventing or treating cancer expressing EGFR exon 20 insertion mutant gene or protein, comprising a step of administering the composition to a subject in need thereof.

The pharmaceutical composition according to one embodiment of the present invention is formulated and used in the form of oral formulations such as powders, granules, tablets, capsules, suspensions, emulsions, syrups and aerosols, external preparations, suppositories or sterile injection solutions according to conventional methods.

In one aspect of the present invention, the active ingredient can be contained in the range of 0.00001 to 100 weight%, 0.0001 to 95 weight% or 0.001 to 90 weight% based on the total weight of the pharmaceutical composition for preventing, improving or treating cancer expressing EGFR exon 20 insertion mutant gene or protein.

In the pharmaceutical composition according to one embodiment of the present invention, the dosage of the compound represented by Formula I or a pharmaceutically acceptable salt thereof may be appropriately changed according to the patient's age, body weight, symptoms, route of administration, and the like.

The dosage of the compound represented by Formula I of the present invention or a pharmaceutically acceptable salt thereof may be 0.00001mg/kg/day to 2000mg/kg/day, 0.0001mg/kg/day to 1000mg/kg/day, 0.001mg/kg/day to 800mg/kg/day, 0.001mg/kg/day to 500mg/kg/day, 0.001mg/kg/day to 100mg/kg/day, 0.001mg/kg/day to 80mg/kg/day, or 0.01mg/kg/day to 70mg/kg/day.

The content of the pyrimidine derivative represented by Formula I of the present invention or a pharmaceutically acceptable salt thereof may be 0.00001 to 100 weight%, 0.0001 to 95 weight%, 0.0001 to 90 weight%, 0.001 to 70 weight%, or 0.001 to 50 weight% per unit dosage form.

The dosage concentration of the compound of Formula I of the present invention or a pharmaceutically acceptable salt thereof may be 0.0001 to 500 µM, 0.001 to 300 µM, 0.001 to 150 µM, 0.001 to 130 µM, 0.001 to 100 µM, 0.001 to 80 µM or 0.01 to 70 µM.

Another embodiment of the present invention relates to the compound represented by Formula I, a solvate, a stereoisomer or a pharmaceutically acceptable salt thereof for use in preventing or treating a disease in which tyrosine kinase domain mutant EGFR is over-expressed.

Another embodiment of the present invention relates to the use of the compound represented by Formula I, a solvate, a stereoisomer or a pharmaceutically acceptable salt thereof for the prevention or treatment of a disease in which tyrosine kinase domain mutant EGFR is over-expressed.

Another example of the present invention relates to the use of the compound represented by Formula I, a solvate, a stereoisomer or a pharmaceutically acceptable salt thereof for preparing a drug for preventing or treating a disease in which tyrosine kinase domain mutant EGFR is over-expressed.

Hereinafter, the present invention will be described in more detail through the Examples. These Examples are for explaining the present invention in more detail, and it is obvious to those skilled in the art that the scope of the present invention is not limited by these Examples according to the gist of the present invention.

The pyrimidine derivative compound represented by Formula I of the present invention may be prepared by the method illustrated in the following reaction scheme, but is not limited thereto.

### Synthesis method of compounds represented by Formula I

The compounds represented by Formula I according to the present invention can be prepared, for example, by referring to the method represented by the following reaction scheme:

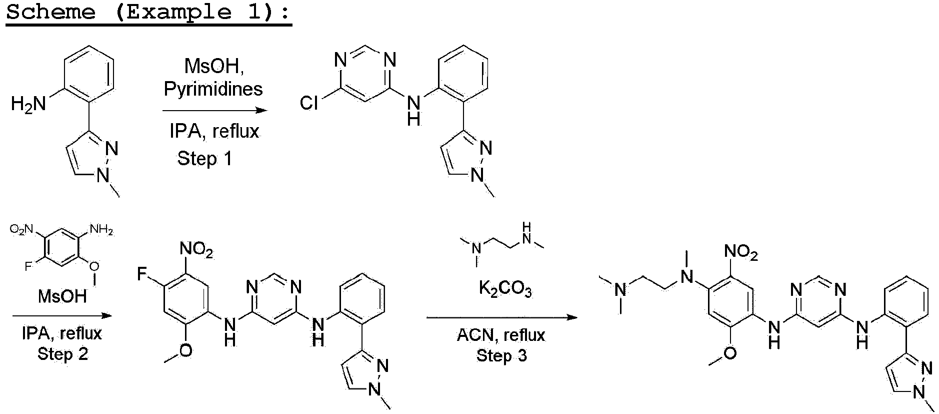

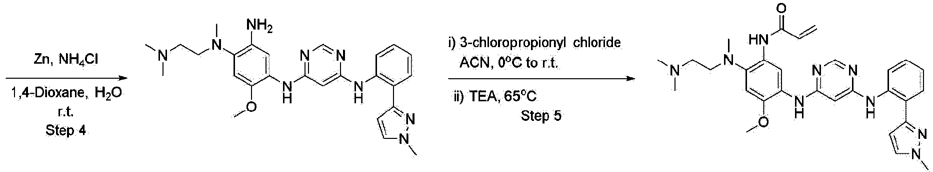

### Step 1: Synthesis of 6-chloro-N-(2-(1-methyl-1H-pyrazol-3-yl)phenyl)pyrimidin-4-amine

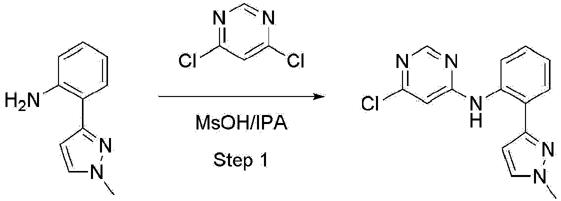

A pyrimidine derivative compound(1.0 equiv.) is dissolved in isopropyl alcohol, and an aniline derivative(1.0 equiv.) and methanesulfonyl acid(1.3 equiv.) are added thereto at room temperature, followed by stirring overnight at 80°C. After completion of the reaction, the solvent is removed by evaporation under reduced pressure and extraction is performed using water and ethyl acetate. The organic layer is evaporated under reduced pressure and subjected to column chromatography to obtain the target compound.

### Step 2: Synthesis of N4-(4-fluoro-2-methoxy-5-nitrophenyl)-N6-(2-(1-methyl-1H-pyrazol-3-yl)phenyl)pyrimidin-4,6-diamine

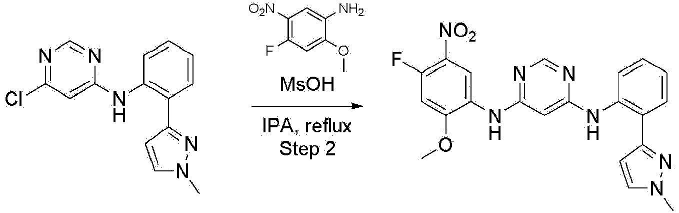

The pyrimidine derivative compound(1.0 equiv.) is dissolved in isopropyl alcohol, and an aniline derivative(1.0 equiv.) and methanesulfonyl acid(1.3 equiv.) are added thereto at room temperature, followed by stirring overnight at 80°C. After completion of the reaction, the solvent is removed by evaporation under reduced pressure, and extraction is performed using water and ethyl acetate. The organic layer is evaporated under reduced pressure and subjected to column chromatography to obtain the target compound.

### Step 3: Synthesis of N4-(4-((2-(dimethylamino)ethyl)(methyl)amino)-2-methoxy-5-nitrophenyl)-N6-(2-(1-methyl-1H-pyrazol-3-yl)phenyl)pyrimidin-4,6-diamine

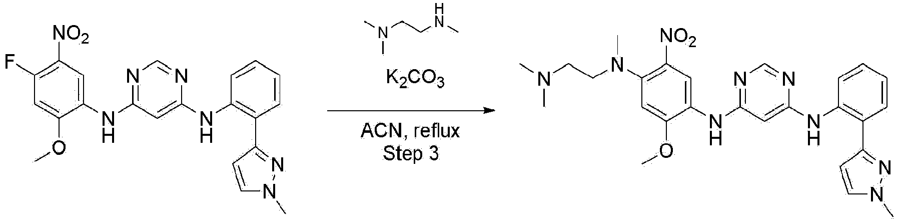

N4-(4-fluoro-2-methoxy-5-nitrophenyl)-N6-(2-(1-methyl-1H-pyrazol-3-yl)phenyl)pyrimidin-4,6-diamine(1.0 equiv.) is dissolved in acetonitrile, and potassium carbonate(3.0 equiv.) and amine chain(1.2 equiv.) are added thereto at room temperature, followed by refluxing and stirring overnight. After completion of the reaction, the temperature was lowered to room temperature and filtration is performed. The filtrate is evaporated under reduced pressure and subjected to column chromatography to obtain the target compound (methylene chloride: methanol = 10:1).

### Step 4: Synthesis of N1-(2-(dimethylamino)ethyl)-5-methoxy-N1-methyl-N4-(6-((2-(1-methyl-1H-pyrazol-3-yl)phenyl)amino)pyrimidin-4-yl)benzene-1,2,4-triamine

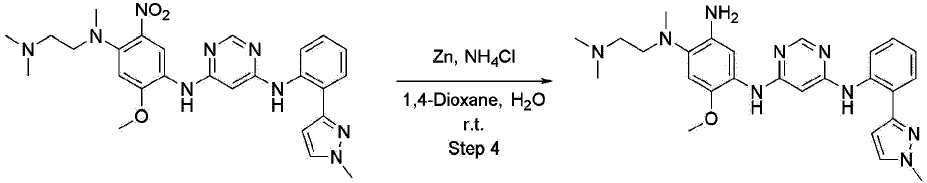

N4-(4-((2-(dimethylamino)ethyl)(methyl)amino)-2-methoxy-5-nitrophenyl)-N6-(2-(1-methyl-1H-pyrazol-3-yl)phenyl)pyrimidin-4,6-diamine(1.0 equiv.) is dissolved in 1,4-dioxane, and zinc(10.0 equiv.) and ammonium chloride(10.0 equiv.) are added thereto at room temperature, followed by stirring overnight. After completion of the reaction, the temperature was lowered to room temperature, and the filtrate was extracted using water and ethyl acetate after filtering with celite. The organic layer is collected, dried, and then evaporated under reduced pressure to obtain a compound. It is used in the next reaction without separation.

### Step 5: Synthesis of N-(2-((2-(dimethylamino)ethyl)(methyl)amino)-4-methoxy-5-((6-((2-(1-methyl-1H-pyrazol-3-yl)phenyl)amino)pyrimidin-4-yl)amino)phenyl)acrylamide

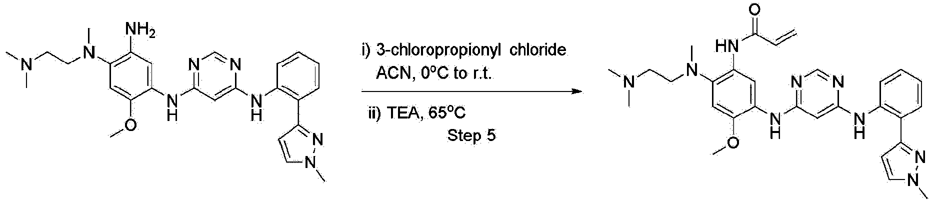

N1-(2-(dimethylamino)ethyl)-5-methoxy-N1-methyl-N4-(6-((2-(1-methyl-1H-pyrazol-3-yl)phenyl)amino)pyrimidin-4-yl)benzene-1,2,4-triamine(1.0 equiv.) is dissolved in tetrahydrofuran and water, and 3-chloropropionyl chloride(1.2 equiv.) is added thereto at 0±5°C, followed by stirring for 15 minutes at the same temperature. After completion of the reaction, sodium hydroxide(4.0 equiv.) is added at the same temperature. The reactor temperature is raised and stirred overnight at 65°C. After completion of the reaction, the solvent is removed by evaporation under reduced pressure, and extraction is performed using water and methylene chloride. The organic layer was evaporated under reduced pressure and subjected to column chromatography to obtain the target compound (methylene chloride: methanol = 10:1).

### Example 1. Preparation of N-(2-((2-(dimethylamino)ethyl)(methyl)amino)-4-methoxy-5-((6-((2-(1-methyl-1H-pyrazol-3-yl)phenyl)amino)pyrimidin-4-yl)amino)phenyl)acrylamide

Yield : 32.0%, White solid, 1H NMR(400 MHz, DMSO-d6) δ 10.30(s, 1H), 10.08(s, 1H), 8.51(s, 1H), 8.31(s, 1H), 8.16(d, J = 0.9 Hz, 1H), 8.08(dd, J = 8.3, 1.2 Hz, 1H), 7.78(d, J = 2.3 Hz, 1H), 7.69(dd, J = 7.8, 1.6 Hz, 1H), 7.20(ddd, J = 8.5, 7.2, 1.6 Hz, 1H), 6.99(td, J = 7.5, 1.2 Hz, 1H), 6.95(s, 1H), 6.71(d, J = 2.4 Hz, 1H), 6.34(dd, J = 16.9, 10.0 Hz, 1H), 6.18(dd, J = 17.0, 2.1 Hz, 1H), 6.14 (d, J = 1.1 Hz, 1H), 5.74 - 5.67(m, 1H), 3.88(s, 3H), 3.76(s, 3H), 2.81(t, J = 5.8 Hz, 2H), 2.66(s, 3H), 2.26(q, J = 5.9 Hz, 2H), 2.16(s, 6H). MS: ESI m/z 542.1 [M+H]+

### Example 2, Preparation of N-(5-((6-((2-(1H-pyrazol-1-yl)phenyl)amino)pyrimidin-4-yl)amino)-2-((2-(dimethylamino)ethyl)(methyl)amino)-4-methoxyphenyl)acrylamide

In Step 1, 2-(1H-pyrazol-1-yl)aniline is used, and the experiment uses the method of Example 1.

Yield : 35.0%, White solid, 1H NMR(400 MHz, DMSO-d6) δ 10.05(s, 1H), 8.97(s, 1H), 8.43(s, 1H), 8.30(s, 1H), 8.10(d, J = 2.4 Hz, 1H), 8.03(d, J = 0.9 Hz, 1H), 7.75(d, J = 1.9 Hz, 1H), 7.73 (dd, J = 8.1, 1.4 Hz, 1H), 7.50 (dd, J = 8.0, 1.5 Hz, 1H), 7.32 (td, J = 7.7, 1.6 Hz, 1H), 7.18(td, J = 7.7, 1.4 Hz, 1H), 6.92 (s, 1H), 6.47 (t, J = 2.2 Hz, 1H), 6.33 (dd, J = 16.9, 10.0 Hz, 1H), 6.18(dd, J = 17.0, 2.2 Hz, 1H), 5.93(s, 1H), 5.72 - 5.68(m, 1H), 3.72(s, 3H), 2.79(t, J = 5.8 Hz, 2H), 2.64(s, 3H), 2.24 (t, J = 5.8 Hz, 2H), 2.15 (s, 6H). MS: ESI m/z 528.2835 [M+H]+

### Example 3. Preparation of N-(2-((2-(dimethylamino)ethyl)(methyl)amino)-4-isopropoxy-5-((6-((2-(1-methyl-1H-pyrazol-3-yl)phenyl)amino)pyrimidin-4-yl)amino)phenyl)acrylamide

In Step 2, 4-fluoro-2-isopropoxy-5-nitroaniline is used, and the experiment uses the method of Example 1.

Yield : 19.6%, White solid, 1H NMR(400 MHz, DMSO-d6) δ 10.30(s, 1H), 10.08(s, 1H), 8.51(s, 1H), 8.31(s, 1H), 8.16(d, J = 0.9 Hz, 1H), 8.08(dd, J = 8.3, 1.2 Hz, 1H), 7.78(d, J = 2.3 Hz, 1H), 7.69(dd, J = 7.8, 1.6 Hz, 1H), 7.20(ddd, J = 8.5, 7.2, 1.6 Hz, 1H), 6.99(td, J = 7.5, 1.2 Hz, 1H), 6.95(s, 1H), 6.71(d, J = 2.4 Hz, 1H), 6.34(dd, J = 16.9, 10.0 Hz, 1H), 6.18(dd, J = 17.0, 2.1 Hz, 1H), 6.14(d, J = 1.1 Hz, 1H), 5.74 - 5.67(m, 1H), 4.64(hept, J = 6.8 Hz, 1H), 3.76(s, 3H), 2.81(t, J = 5.8 Hz, 2H), 2.66(s, 3H), 2.26(q, J = 5.9 Hz, 2H), 2.16(s, 6H), 1.28(d, J = 6.8 Hz, 6H). MS: ESI m/z 570.1 [M+H]+

### Example 4. Preparation of N-(4-methoxy-5-((6-((2-(1-methyl-1H-pyrazol-3-yl)phenyl)amino)pyrimidin-4-yl)amino)-2-(4-methylpiperizin-1-yl)phenyl)acrylamide

The method of Example 1 is used and in step 3, 1-methylpiperizine is used.

Yield : 23.5%, Off-white solid, 1H NMR(400 MHz, DMSO-d6) δ 10.30(s, 1H), 10.08(s, 1H), 8.51(s, 1H), 8.31(s, 1H), 8.16(d, J = 0.9 Hz, 1H), 8.08(dd, J = 8.3, 1.2 Hz, 1H), 7.78(d, J = 2.3 Hz, 1H), 7.69(dd, J = 7.8, 1.6 Hz, 1H), 7.20(ddd, J = 8.5, 7.2, 1.6 Hz, 1H), 6.99(td, J = 7.5, 1.2 Hz, 1H), 6.95(s, 1H), 6.71(d, J = 2.4 Hz, 1H), 6.34 (dd, J = 16.9, 10.0 Hz, 1H), 6.18(dd, J = 17.0, 2.1 Hz, 1H), 6.14(d, J = 1.1 Hz, 1H), 5.74 - 5.67(m, 1H), 3.88(s, 3H), 3.76(s, 3H), 2.83 - 2.80(m, 4H), 2.42(m, 4H), 2.20(s, 3H). MS: ESI m/z 540.0 [M+H]+

### Example 5. Preparation of N-(4-methoxy-5-((6-((2-(1-methyl-1H-pyrazol-3-yl)phenyl)amino)pyrimidin-4-yl)amino)-2-morpholinophenyl)acrylamide

The method of Example 1 is used and in step 3, morpholine is used.

Yield : 22.1%, Off-white solid, 1H NMR(400 MHz, DMSO-d6) δ 10.30(s, 1H), 10.08(s, 1H), 8.51(s, 1H), 8.31(s, 1H), 8.16(d, J = 0.9 Hz, 1H), 8.08(dd, J = 8.3, 1.2 Hz, 1H), 7.78(d, J = 2.3 Hz, 1H), 7.69(dd, J = 7.8, 1.6 Hz, 1H), 7.20(ddd, J = 8.5, 7.2, 1.6 Hz, 1H), 6.99(td, J = 7.5, 1.2 Hz, 1H), 6.95(s, 1H), 6.71(d, J = 2.4 Hz, 1H), 6.34(dd, J = 16.9, 10.0 Hz, 1H), 6.18(dd, J = 17.0, 2.1 Hz, 1H), 6.14 (d, J = 1.1 Hz, 1H), 5.74 - 5.67(m, 1H), 3.88(s, 3H), 3.76(s, 3H), 3.71 - 3.68(m, 4H), 2.81 - 2.79(m, 4H). MS: ESI m/z 527.0 [M+H]+

### Example 6. Preparation of (S)-N-(2-(3-(dimethylamino)pyrrolidin-1-yl)-4-methoxy-5-((6-((2-(1-methyl-1H-pyrazol-3-yl)phenyl)amino)pyrimidin-4-yl)amino)phenyl)acrylamide

The method of Example 1 is used and in step 3, (S)-N,N-dimethylpyrrolidin-3-amine is used.

Yield : 18.4%, Off-white solid, 1H NMR(400 MHz, DMSO-d6) δ 10.30(s, 1H), 10.08(s, 1H), 8.51(s, 1H), 8.31(s, 1H), 8.16(d, J = 0.9 Hz, 1H), 8.08(dd, J = 8.3, 1.2 Hz, 1H), 7.78(d, J = 2.3 Hz, 1H), 7.69(dd, J = 7.8, 1.6 Hz, 1H), 7.20(ddd, J = 8.5, 7.2, 1.6 Hz, 1H), 6.99(td, J = 7.5, 1.2 Hz, 1H), 6.95(s, 1H), 6.71(d, J = 2.4 Hz, 1H), 6.34 (dd, J = 16.9, 10.0 Hz, 1H), 6.18(dd, J = 17.0, 2.1 Hz, 1H), 6.14(d, J = 1.1 Hz, 1H), 5.74 - 5.67(m, 1H), 3.88(s, 3H), 3.76(s, 3H), 3.37 - 3.30(m, 1H), 3.17 - 3.13(m, 3H), 2.64 - 2.57(m, 1H), 2.11(s, 6H), 2.05 - 1.99(m, 1H), 1.70 - 1.61(m, 1H). MS: ESI m/z 534.1 [M+H]+

### Example 7. Preparation of (R)-N-(2-(3-(dimethylamino)pyrrolidin-1-yl)-4-methoxy-5-((6-((2-(1-methyl-1H-pyrazol-3-yl)phenyl)amino)pyrimidin-4-yl)amino)phenyl)acrylamide

The method of Example 1 is used and in step 3, (R)-N,N-dimethylpyrrolidin-3-amine is used.

Yield : 17.8%, Off-white solid, 1H NMR(400 MHz, DMSO-d6) δ 10.30(s, 1H), 10.08(s, 1H), 8.51(s, 1H), 8.31(s, 1H), 8.16(d, J = 0.9 Hz, 1H), 8.08(dd, J = 8.3, 1.2 Hz, 1H), 7.78(d, J = 2.3 Hz, 1H), 7.69(dd, J = 7.8, 1.6 Hz, 1H), 7.20(ddd, J = 8.5, 7.2, 1.6 Hz, 1H), 6.99(td, J = 7.5, 1.2 Hz, 1H), 6.95(s, 1H), 6.71(d, J = 2.4 Hz, 1H), 6.34(dd, J = 16.9, 10.0 Hz, 1H), 6.18(dd, J = 17.0, 2.1 Hz, 1H), 6.14 (d, J = 1.1 Hz, 1H), 5.74 - 5.67(m, 1H), 3.88(s, 3H), 3.76(s, 3H), 3.37 - 3.30(m, 1H), 3.17 - 3.13(m, 3H), 2.64 - 2.57(m, 1H), 2.11(s, 6H), 2.05 - 1.99(m, 1H), 1.70 - 1.61(m, 1H). MS: ESI m/z 534.1 [M+H]+

### Example 8. Preparation of (R)-N-(4-methoxy-2-(methyl(1-methylpyrrolidin-3-yl)amino)-5-((6-((2-(1-methyl-1H-pyrazol-3-yl)phenyl)amino)pyrimidin-4-yl)amino)phenyl)acrylamide

The method of Example 1 is used and in step 3, (R)-N,1-dimethylpyrrolidin-3-amine is used.

Yield : 22.2%, Off-white solid, 1H NMR(400 MHz, DMSO-d6) δ 10.30(s, 1H), 10.08(s, 1H), 8.51(s, 1H), 8.31(s, 1H), 8.16(d, J = 0.9 Hz, 1H), 8.08(dd, J = 8.3, 1.2 Hz, 1H), 7.78(d, J = 2.3 Hz, 1H), 7.69(dd, J = 7.8, 1.6 Hz, 1H), 7.20(ddd, J = 8.5, 7.2, 1.6 Hz, 1H), 6.99(td, J = 7.5, 1.2 Hz, 1H), 6.95(s, 1H), 6.71(d, J = 2.4 Hz, 1H), 6.34 (dd, J = 16.9, 10.0 Hz, 1H), 6.18(dd, J = 17.0, 2.1 Hz, 1H), 6.14(d, J = 1.1 Hz, 1H), 5.74 - 5.67(m, 1H), 3.88(s, 3H), 3.76(s, 3H), 3.58 - 3.51(m, 1H), 2.54 - 2.49(m, 4H), 2.45 - 2.42(m, 1H), 2.39 - 2.33(m, 2H), 1.90 - 1.81(m, 1H), 1.71 - 1.63(m, 1H). MS: ESI m/z 534.1 [M+H]+

### Example 9. Preparation of (S)-N-(4-methoxy-2-(methyl(1-methylpyrrolidin-3-yl)amino)-5-((6-((2-(1-methyl-1H-pyrazol-3-yl)phenyl)amino)pyrimidin-4-yl)amino)phenyl)acrylamide

The method of Example 1 is used and in step 3, (S)-N,1-dimethylpyrrolidin-3-amine is used.

Yield : 20.1%, Off-white solid, 1H NMR(400 MHz, DMSO-d6) δ 10.30(s, 1H), 10.08(s, 1H), 8.51(s, 1H), 8.31(s, 1H), 8.16(d, J = 0.9 Hz, 1H), 8.08(dd, J = 8.3, 1.2 Hz, 1H), 7.78(d, J = 2.3 Hz, 1H), 7.69(dd, J = 7.8, 1.6 Hz, 1H), 7.20(ddd, J = 8.5, 7.2, 1.6 Hz, 1H), 6.99(td, J = 7.5, 1.2 Hz, 1H), 6.95(s, 1H), 6.71(d, J = 2.4 Hz, 1H), 6.34(dd, J = 16.9, 10.0 Hz, 1H), 6.18(dd, J = 17.0, 2.1 Hz, 1H), 6.14 (d, J = 1.1 Hz, 1H), 5.74 - 5.67(m, 1H), 3.88(s, 3H), 3.76(s, 3H), 3.58 - 3.51(m, 1H), 2.54 - 2.49(m, 4H), 2.45 - 2.42(m, 1H), 2.39 - 2.33(m, 2H), 1.90 - 1.81(m, 1H), 1.71 - 1.63(m, 1H). MS: ESI m/z 534.1 [M+H]+

### Example 10. Preparation of N-(2-((2-(diamethylamino)ethyl)(methyl)amino)-5-((6-((2-(1-methyl-1H-pyrazol-3-yl)phenyl)amino)pyrimidin-4-yl)amino)-4-(2,2,2-trifluoroethoxy)phenyl)acrylamide

In Step 2, 4-fluoro-5-nitro-2-(2,2,2-trifluoroethoxy)aniline is used, and the experiment uses the method of Example 1.

Yield : 21.2%, Off-white solid, 1H NMR(400 MHz, DMSO-d6) δ 10.32(s, 1H), 10.06(s, 1H), 8.47(s, 1H), 8.29(s, 1H), 8.16(d, J = 0.9 Hz, 1H), 8.08(dd, J = 8.3, 1.2 Hz, 1H), 7.78(d, J = 2.3 Hz, 1H), 7.69(dd, J = 7.8, 1.6 Hz, 1H), 7.20(ddd, J = 8.5, 7.2, 1.6 Hz, 1H), 6.99(td, J = 7.5, 1.2 Hz, 1H), 6.95(s, 1H), 6.71(d, J = 2.4 Hz, 1H), 6.34 (dd, J = 16.9, 10.0 Hz, 1H), 6.18(dd, J = 17.0, 2.1 Hz, 1H), 6.14(d, J = 1.1 Hz, 1H), 5.74 - 5.67(m, 1H), 4.61 - 4.59(m, 2H), 3.76 (s, 3H), 2.81(t, J = 5.8 Hz, 2H), 2.66(s, 3H), 2.26(q, J = 5.9 Hz, 2H), 2.17(s, 6H). MS: ESI m/z 610.1 [M+H] +

### Example 11. Preparation of N-(2-((2-(dimethylamino)ethyl)(methyl)amino)-4-ethoxy-5-((6-((2-(1-methyl-1H-pyrazol-3-yl)phenyl)amino)pyrimidin-4-yl)amino)phenyl)acetamide

In Step 2, 2-ethoxy-4-fluoro-5-nitroaniline is used, and the experiment uses the method of Example 1.

Yield : 31.2%, Off-white solid, 1H NMR(400 MHz, DMSO-d6) δ 10.32(s, 1H), 10.07(s, 1H), 8.51(s, 1H), 8.31(s, 1H), 8.16(d, J = 0.9 Hz, 1H), 8.08(dd, J = 8.3, 1.2 Hz, 1H), 7.78(d, J = 2.3 Hz, 1H), 7.69(dd, J = 7.8, 1.6 Hz, 1H), 7.20(ddd, J = 8.5, 7.2, 1.6 Hz, 1H), 6.99(td, J = 7.5, 1.2 Hz, 1H), 6.95(s, 1H), 6.71(d, J = 2.4 Hz, 1H), 6.34(dd, J = 16.9, 10.0 Hz, 1H), 6.18(dd, J = 17.0, 2.1 Hz, 1H), 6.14 (d, J = 1.1 Hz, 1H), 5.74 - 5.67(m, 1H), 4.10(q, J = 7.8 Hz, 2H), 3.76(s, 3H), 2.81(t, J = 5.8 Hz, 2H), 2.66(s, 3H), 2.26(q, J = 5.9 Hz, 2H), 2.16(s, 6H), 1.40(t, J = 7.9 Hz, 3H). MS: ESI m/z 556.1 [M+H]+

### Example 12. Preparation of N-(4-cyclopropoxy-2-((2-(dimethylamino)ethyl)(methyl)amino)-5-((6-((2-(1-methyl-1H-pyrazol-3-yl)phenyl)amino)pyrimidin-4-yl)amino)phenyl)acrylamide

In Step 2, 2-cyclopropoxy-4-fluoro-5-nitroaniline is used, and the experiment uses the method of Example 1.

Yield : 29.3%, Off-white solid, 1H NMR(400 MHz, DMSO-d6) δ 10.31(s, 1H), 10.06(s, 1H), 8.51(s, 1H), 8.31(s, 1H), 8.16(d, J = 0.9 Hz, 1H), 8.08(dd, J = 8.3, 1.2 Hz, 1H), 7.78(d, J = 2.3 Hz, 1H), 7.69(dd, J = 7.8, 1.6 Hz, 1H), 7.20(ddd, J = 8.5, 7.2, 1.6 Hz, 1H), 6.99(td, J = 7.5, 1.2 Hz, 1H), 6.95(s, 1H), 6.71(d, J = 2.4 Hz, 1H), 6.34 (dd, J = 16.9, 10.0 Hz, 1H), 6.18(dd, J = 17.0, 2.1 Hz, 1H), 6.14(d, J = 1.1 Hz, 1H), 5.74 - 5.67(m, 1H), 3.76(s, 3H), 3.42 - 3.33(m, 1H), 2.81(t, J = 5.8 Hz, 2H), 2.66(s, 3H), 2.26(q, J = 5.9 Hz, 2H), 2.16(s, 6H), 0.60 - 0.57(m, 2H), 0.37 - 0.34(m, 2H). MS: ESI m/z 568.1 [M+H]+

### Example 13. Preparation of N-(5-((5-cyano-4-((2-(1-methyl-1H-pyrazol-3-yl)phenyl)amino)pyrimidin-2-yl)amino)-2-((2-(dimethylamino)ethyl)(methyl)amino)-4-methoxyphenyl)acrylamide

In Step 1-1, 2,4-dichloropyrimidin-5-carbonitrile is used, and the experiment uses the method of Example 1.

Yield : 19.5%, Off-white solid, 1H NMR(400 MHz, DMSO-d6) δ 11.34(s, 1H), 10.07(s, 1H), 8.57(d, J = 8.1 Hz, 1H), 8.39(s, 1H), 8.37(s, 1H), 8.33(s, 1H), 7.82(d, J = 2.3 Hz, 1H), 7.68(dd, J = 7.6, 1.9 Hz, 1H), 7.06 - 6.92(m, 3H), 6.76(d, J = 2.4 Hz, 1H), 6.35(dd, J = 16.9, 10.1 Hz, 1H), 6.13(dd, J = 17.0, 2.0 Hz, 1H), 5.68(dd, J = 10.1, 2.1 Hz, 1H), 3.91(s, 3H), 3.72(s, 3H), 2.84(t, J = 5.9 Hz, 2H), 2.69(s, 3H), 2.28(t, J = 5.7 Hz, 2H), 2.17(s, 6H). MS: ESI m/z 567.1 [M+H]+

### Example 14. Preparation of N-(5-((4-((2-(1H-pyrazol-1-yl)phenyl)amino)-5-methylpyrimidin-2-yl)amino)-2-((2-(dimethylamino)ethyl)(methyl)amino)-4-methoxyphenyl)acrylamide

In Step 1-1, 2-(1H-pyrazol-1-yl)aniline, 2,4-dichloro-5-methylpyrimidine are used, and the experiment uses the method of Example 1.

Yield : 21.3%, Off-white solid, 1H NMR(400 MHz, DMSO-D6) δ 10.05(s, 1H), 9.89(s, 1H), 8.34(d, J = 14.0 Hz, 2H), 8.29(s, 1H), 8.24 (d, J = 2.4 Hz, 1H), 7. 94 (s, 1H), 7.88 (d, J = 1.8 Hz, 1H), 7.49(dd, J = 7.5, 2.0 Hz, 1H), 7.16 - 7.04(m, 2H), 6.96(s, 1H), 6.58 - 6.53(m, 1H), 6.35(dd, J = 16.9, 10.1 Hz, 1H), 6.15(dd, J = 16.9, 2.1 Hz, 1H), 5.74 - 5.66(m, 1H), 3.72(s, 3H), 2.83(t, J = 5.9 Hz, 2H), 2.68(s, 3H), 2.26(t, J = 5.9 Hz, 2H), 2.16(s, 6H), 2.09(s, 3H). MS: ESI m/z 542.1 [M+H]+

### Example 15. Preparation of N-(2-((2-(dimethylamino)ethyl)(methyl)amino)-4-methoxy-5-((4-((2-(1-methyl-1H-pyrazol-3-yl)phenyl)amino)-5-(trifluoromethyl)pyrimidin-2-yl)amino)phenyl)acrylamide

In Step 1-1, 2,4-dichloro-5-(trifluoromethyl)pyrimidine is used, and the experiment uses the method of Example 1.

Yield : 17.5%, Off-white solid, 1H NMR(400 MHz, DMSO-d6) δ 11.34(s, 1H), 10.58(s, 1H), 8.76(s, 1H), 8.57(d, J = 8.1 Hz, 1H), 8.39(s, 1H), 8.33(s, 1H), 7.82(d, J = 2.3 Hz, 1H), 7.68(dd, J = 7.6, 1.9 Hz, 1H), 7.06 - 6.92(m, 3H), 6.76(d, J = 2.4 Hz, 1H), 6.35(dd, J = 16.9, 10.1 Hz, 1H), 6.13(dd, J = 17.0, 2.0 Hz, 1H), 5.68(dd, J = 10.1, 2.1 Hz, 1H), 3.91(s, 3H), 3.72(s, 3H), 2.84(t, J = 5.9 Hz, 2H), 2.69(s, 3H), 2.28(t, J = 5.7 Hz, 2H), 2.17(s, 6H). MS: ESI m/z 610.0 [M+H]+

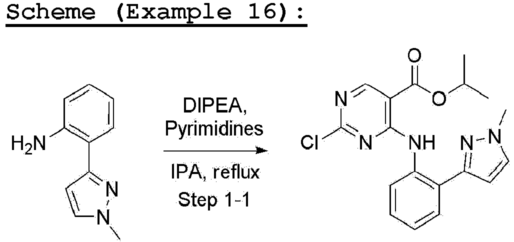

### Step 1-1: Synthesis of isopropyl 2-chloro-4-((2-(1-methyl-1H-pyrazol-3-yl)phenyl)amino)pyrimidin-5-carboxylate

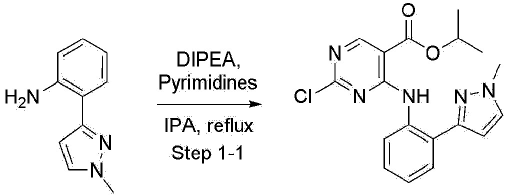

An aniline derivative(1.0 equiv.) is dissolved in isopropyl alcohol, and isopropyl 2,4-dichloropyrimidin-5-carboxylate(1.1 equiv.) and N,N-diisopropylethylamine(2.5 equiv.) are added thereto at room temperature, followed by stirring overnight at 80°C. After completion of the reaction, it was evaporated under reduced pressure and extraction is performed using water and dichloromethane. The organic layer is washed with 2N hydrochloric acid. The organic layer is evaporated under reduced pressure and subjected to column chromatography to obtain the target compound.

### Example 16. Preparation of isopropyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate

Except for Step 1-1, all experiments use the method of Example 1.

Yield : 19.4%, Off-white solid, 1H NMR(400 MHz, DMSO-d6) δ 11.40(s, 1H), 9.90(s, 1H), 8.76(s, 1H), 8.60(s, 1H), 8.23(s, 1H), 8.16(s,1H), 7.73(d, J = 2.3 Hz, 1H), 7.46(d, J = 7.1 Hz, 1H), 6.94(s, 3H), 6.48(d, J = 2.3 Hz, 1H), 6.18(d, J = 16.7 Hz, 1H), 5.71(dd, J = 10.3, 1.8 Hz, 1H), 5.07(hept, J = 6.1 Hz, 1H), 3.88(s, 3H), 3.72(s, 3H), 2.92(m, 2H), 2.64(s, 3H), 2.28(m, 6H), 1.27(d, J = 6.2 Hz, 6H). MS: ESI m/z 628.2 [M+H]+

### Example 17. Preparation of isopropyl 4-((2-(1H-pyrazol-1-yl)phenyl)amino)-2-((5-acrylamido-4-((2-(dimethylamino)ethyl)(methyl)amino)-2-methoxyphenyl)amino)pyrimidin-5-carboxylate

In Step 1-1, 2-(1H-pyrazol-1-yl)aniline is used, and all experiments except Step 1-1 use the method of Example 1.

Yield : 20.3%, Off-white solid, 1H NMR(400 MHz, DMSO-d6) δ 10.56(s, 1H), 10.06(s, 1H), 8.91(s, 1H), 8.56(s, 1H), 8.26(s, 1H), 8.08(s, 1H), 8.00(d, J = 2.4 Hz, 1H), 7.73(dd, J = 1.8, 0.6 Hz, 1H), 7.30(d, J = 7.5 Hz, 1H), 7.06(s, 2H), 6.97(s, 1H), 6.50 - 6.44(m, 1H), 6.37(dd, J = 16.8, 10.1 Hz, 1H), 6.17(dd, J = 16.9, 2.1 Hz, 1H), 5.71(dd, J = 10.1, 2.1 Hz, 1H), 5.00(hept, J = 6.3 Hz, 1H), 3.71(s, 3H), 2.84(s, 2H), 2.68(s, 3H), 2.19(s, 8H), 1.24(d, J = 6.2 Hz, 6H). MS: ESI m/z 614.1 [M+H]+

### Example 18. Preparation of methyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate

In Step 1-1, methyl 2,4-dichloropyrimidin-5-carboxylate is used, and all experiments use the method of Example 1.

Yield : 16.7%, Off-white solid, 1H NMR(400 MHz, DMSO-d6) δ 11.40(s, 1H), 9.90(s, 1H), 8.76(s, 1H), 8.60(s, 1H), 8.23(s, 1H), 8.16(s,1H), 7.73(d, J = 2.3 Hz, 1H), 7.46(d, J = 7.1 Hz, 1H), 6.94(s, 3H), 6.48(d, J = 2.3 Hz, 1H), 6.18(d, J = 16.7 Hz, 1H), 5.71(dd, J = 10.3, 1.8 Hz, 1H), 3.92(s, 3H), 3.88(s, 3H), 3.72(s, 3H), 2.92(m, 2H), 2.64(s, 3H), 2.28(m, 8H). MS: ESI m/z 600.1 [M+H]+

### Example 19. Preparation of methyl 4-((2-(1H-pyrazol-1-yl)phenyl)amino)-2-((5-acrylamido-4-((2-(dimethylamino)ethyl)(methyl)amino)-2-methoxyphenyl)amino)pyrimidin-5-carboxylate

In Step 1-1, 2-(1H-pyrazol-1-yl)aniline and methyl 2,4-dichloropyrimidin-5-carboxylate are used, and all experiments use the method of Example 1.

Yield : 17.4%, Off-white solid, 1H NMR(400 MHz, DMSO-d6) δ 10.56(s, 1H), 10.06(s, 1H), 8.91(s, 1H), 8.56(s, 1H), 8.26(s, 1H), 8.08(s, 1H), 8.00(d, J = 2.4 Hz, 1H), 7.73(dd, J = 1.8, 0.6 Hz, 1H), 7.30(d, J = 7.5 Hz, 1H), 7.06(s, 2H), 6.97(s, 1H), 6.50 - 6.44(m, 1H), 6.37(dd, J = 16.8, 10.1 Hz, 1H), 6.17(dd, J = 16.9, 2.1 Hz, 1H), 5.71(dd, J = 10.1, 2.1 Hz, 1H), 3.92(s, 3H), 3.71(s, 3H), 2.84(s, 2H), 2.68(s, 3H), 2.29(s, 2H), 2.19(s, 6H). MS: ESI m/z 586.1 [M+H]+

### Example 20. Preparation of ethyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate

In Step 1-1, ethyl 2,4-dichloropyrimidin-5-carboxylate is used, and all experiments use the method of Example 1.

Yield : 19.3%, Off-white solid, 1H NMR(400 MHz, DMSO-d6) δ 11.40(s, 1H), 9.90(s, 1H), 8.76(s, 1H), 8.60(s, 1H), 8.23(s, 1H), 8.16(s,1H), 7.73(d, J = 2.3 Hz, 1H), 7.46(d, J = 7.1 Hz, 1H), 6.94(s, 3H), 6.48(d, J = 2.3 Hz, 1H), 6.18(d, J = 16.7 Hz, 1H), 5.71 (dd, J = 10.3, 1.8 Hz, 1H), 4.38 (q, J = 8.0 Hz, 2H), 3.88(s, 3H), 3.72(s, 3H), 2.92(m, 2H), 2.64(s, 3H), 2.28(m, 8H), 1.34(t, J = 8.0 Hz, 3H). MS: ESI m/z 614.1 [M+H]+

### Example 21. Preparation of ethyl 4-((2-(1H-pyrazol-1-yl)phenyl)amino)-2-((5-acrylamido-4-((2-(dimethylamino)ethyl)(methyl)amino)-2-methoxyphenyl)amino)pyrimidin-5-carboxylate

In Step 1-1, 2-(1H-pyrazol-1-yl)aniline and ethyl 2,4-dichloropyrimidin-5-carboxylate are used, and all experiments use the method of Example 1.

Yield : 19.6%, Off-white solid, 1H NMR(400 MHz, DMSO-d6) δ 10.56(s, 1H), 10.06(s, 1H), 8.91(s, 1H), 8.56(s, 1H), 8.26(s, 1H), 8.08(s, 1H), 8.00(d, J = 2.4 Hz, 1H), 7.73(dd, J = 1.8, 0.6 Hz, 1H), 7.30(d, J = 7.5 Hz, 1H), 7.06(s, 2H), 6.97(s, 1H), 6.50 - 6.44(m, 1H), 6.37(dd, J = 16.8, 10.1 Hz, 1H), 6.17(dd, J = 16.9, 2.1 Hz, 1H), 5.71 (dd, J = 10.1, 2.1 Hz, 1H), 4.38(q, J = 8.0 Hz, 2H), 3.71(s, 3H), 2.84(s, 2H), 2.68(s, 3H), 2.29(s, 2H), 2.19(s, 6H), 1.34(t, J = 8.0 Hz, 3H). MS: ESI m/z 600.1 [M+H]+

### Example 22. Preparation of cyclopropyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate

In Step 1-1, cyclopropyl 2,4-dichloropyrimidin-5-carboxylate is used, and all experiments use the method of Example 1.

Yield : 19.3%, Off-white solid, 1H NMR(400 MHz, DMSO-d6) δ 11.40(s, 1H), 9.90(s, 1H), 8.76(s, 1H), 8.60(s, 1H), 8.23(s, 1H), 8.16(s,1H), 7.73(d, J = 2.3 Hz, 1H), 7.46(d, J = 7.1 Hz, 1H), 6.94(s, 3H), 6.48(d, J = 2.3 Hz, 1H), 6.18(d, J = 16.7 Hz, 1H), 5.71(dd, J = 10.3, 1.8 Hz, 1H), 3.88(s, 3H), 3.72(s, 3H), 3.37(m, 1H), 2.92(m, 2H), 2.64(s, 3H), 2.28(m, 6H), 0.44 - 0.39(m, 2H), 0.27 - 0.24(m, 2H). MS: ESI m/z 626.1 [M+H]+

### Example 23. Preparation of isopropyl 2-((5-acrylamido-2-methoxy-4-(4-methylpiperizin-1-yl)phenyl)amino)-4-((2-(1-methyl-1H-pyrazol-3-yl)phenyl)amino)pyrimidin-5-carboxylate

Except for Step 1-1, all experiments use the method of Example 1, and in Step 3, 1-methylpiperizine is used.

Yield : 31.2%, Off-white solid, 1H NMR(400 MHz, DMSO-d6) δ 11.40(s, 1H), 9.90(s, 1H), 8.76(s, 1H), 8.60(s, 1H), 8.23(s, 1H), 8.16(s,1H), 7.73(d, J = 2.3 Hz, 1H), 7.46(d, J = 7.1 Hz, 1H), 6.94(s, 3H), 6.48(d, J = 2.3 Hz, 1H), 6.18(d, J = 16.7 Hz, 1H), 5.71(dd, J = 10.3, 1.8 Hz, 1H), 5.07(h, J = 6.1 Hz, 1H), 3.88(s, 3H), 3.72(s, 3H), 2.83 - 2.80(m, 4H), 2.42(m, 4H), 2.21(s, 3H), 1.27(d, J = 6.2 Hz, 6H). MS: ESI m/z 626.1 [M+H]+

### Example 24. Preparation of isopropyl 2-((5-acrylamido-2-methoxy-4-morpholinophenyl)amino)-4-((2-(1-methyl-1H-pyrazol-3-yl)phenyl)amino)pyrimidin-5-carboxylate

Except for Step 1-1, all experiments use the method of Example 1, and in Step 3, morpholine is used.

Yield : 27.7%, Off-white solid, 1H NMR(400 MHz, DMSO-d6) δ 11.40(s, 1H), 9.90(s, 1H), 8.76(s, 1H), 8.60(s, 1H), 8.23(s, 1H), 8.16(s,1H), 7.73(d, J = 2.3 Hz, 1H), 7.46(d, J = 7.1 Hz, 1H), 6.94(s, 3H), 6.48(d, J = 2.3 Hz, 1H), 6.18(d, J = 16.7 Hz, 1H), 5.71(dd, J = 10.3, 1.8 Hz, 1H), 5.07(h, J = 6.1 Hz, 1H), 3.88(s, 3H), 3.72(s, 3H), 3.71 - 3.68(m, 4H), 2.81 - 2.79(m, 4H), 1.27(d, J = 6.2 Hz, 6H). MS: ESI m/z 613.1 [M+H]+

### Example 25. Preparation of isopropyl(S)-2-((5-acrylamido-4-(3-(dimethylamino)pyrrolidin-1-yl)-2-methoxyphenyl)amino)-4-((2-(1-methyl-1H-pyrazol-3-yl)phenyl)amino)pyrimidin-5-carboxylate

Except for Step 1-1, all experiments use the method of Example 1, and in Step 3, (S)-N,N-dimethylpyrrolidin-3-amine is used.

Yield : 22.3%, Off-white solid, 1H NMR(400 MHz, DMSO-d6) δ 11.39(s, 1H), 9.92(s, 1H), 8.76(s, 1H), 8.60(s, 1H), 8.23(s, 1H), 8.16(s,1H), 7.73(d, J = 2.3 Hz, 1H), 7.46(d, J = 7.1 Hz, 1H), 6.94(s, 3H), 6.48(d, J = 2.3 Hz, 1H), 6.18(d, J = 16.7 Hz, 1H), 5.71(dd, J = 10.3, 1.8 Hz, 1H), 5.07(h, J = 6.1 Hz, 1H), 3.86(s, 3H), 3.71(s, 3H), 3.37 - 3.30(m, 1H), 3.17 - 3.13(m, 3H), 2.64 - 2.57(m, 1H), 2.11(s, 6H), 2.05 - 1.99(m, 1H), 1.70 - 1.61(m, 1H), 1.27(d, J = 6.2 Hz, 6H). MS: ESI m/z 640.1 [M+H]+

### Example 26. Preparation of isopropyl(R)-2-((5-acrylamido-4-(3-(dimethylamino)pyrrolidin-1-yl)-2-methoxyphenyl)amino)-4-((2-(1-methyl-1H-pyrazol-3-yl)phenyl)amino)pyrimidin-5-carboxylate

Except for Step 1-1, all experiments use the method of Example 1, and in Step 3, (R)-N,N-dimethylpyrrolidin-3-amine is used.

Yield : 20.1%, Off-white solid, 1H NMR(400 MHz, DMSO-d6) δ 11.39(s, 1H), 9.92(s, 1H), 8.76(s, 1H), 8.60(s, 1H), 8.23(s, 1H), 8.16(s,1H), 7.73(d, J = 2.3 Hz, 1H), 7.46(d, J = 7.1 Hz, 1H), 6.94(s, 3H), 6.48(d, J = 2.3 Hz, 1H), 6.18(d, J = 16.7 Hz, 1H), 5.71(dd, J = 10.3, 1.8 Hz, 1H), 5.07(h, J = 6.1 Hz, 1H), 3.86(s, 3H), 3.71(s, 3H), 3.37 - 3.30(m, 1H), 3.17 - 3.13(m, 3H), 2.64 - 2.57(m, 1H), 2.11(s, 6H), 2.05 - 1.99(m, 1H), 1.70 - 1.61(m, 1H), 1.27(d, J = 6.2 Hz, 6H). MS: ESI m/z 640.1 [M+H]+

### Example 27. Preparation of isopropyl(R)-2-((5-acrylamido-2-methoxy-4-(methyl(1-methylpyrrolidin-3-yl)amino)phenyl)amino)-4-((2-(1-methyl-1H-pyrazol-3-yl)phenyl)amino)pyrimidin-5-carboxylate

Except for Step 1-1, all experiments use the method of Example 1, and in Step 3, (R)-N,1-dimethylpyrrolidin-3-amine is used.

Yield : 21.1%, Off-white solid, 1H NMR(400 MHz, DMSO-d6) δ 11.41(s, 1H), 9.91(s, 1H), 8.73(s, 1H), 8.60(s, 1H), 8.23(s, 1H), 8.16(s,1H), 7.73(d, J = 2.3 Hz, 1H), 7.46(d, J = 7.1 Hz, 1H), 6.94(s, 3H), 6.48(d, J = 2.3 Hz, 1H), 6.18(d, J = 16.7 Hz, 1H), 5.71(dd, J = 10.3, 1.8 Hz, 1H), 5.07(h, J = 6.1 Hz, 1H), 3.85(s, 3H), 3.70(s, 3H), 3.58 - 3.51(m, 1H), 2.54 - 2.49(m, 4H), 2.45 - 2.42(m, 1H), 2.39 - 2.33(m, 2H), 1.90 - 1.81(m, 1H), 1.71 - 1.63(m, 1H), 1.27(d, J = 6.2 Hz, 6H). MS: ESI m/z 640.1 [M+H]+

### Example 28. Preparation of isopropyl(S)-2-((5-acrylamido-2-methoxy-4-(methyl(1-methylpyrrolidin-3-yl)amino)phenyl)amino)-4-((2-(1-methyl-1H-pyrazol-3-yl)phenyl)amino)pyrimidin-5-carboxylate

Except for Step 1-1, all experiments use the method of Example 1, and in Step 3, (S)-N,1-dimethylpyrrolidin-3-amine is used.

Yield : 18.3%, Off-white solid, 1H NMR(400 MHz, DMSO-d6) δ 11.41(s, 1H), 9.91(s, 1H), 8.73(s, 1H), 8.60(s, 1H), 8.23(s, 1H), 8.16(s,1H), 7.73(d, J = 2.3 Hz, 1H), 7.46(d, J = 7.1 Hz, 1H), 6.94(s, 3H), 6.48(d, J = 2.3 Hz, 1H), 6.18(d, J = 16.7 Hz, 1H), 5.71(dd, J = 10.3, 1.8 Hz, 1H), 5.07(h, J = 6.1 Hz, 1H), 3.85(s, 3H), 3.70(s, 3H), 3.58 - 3.51(m, 1H), 2.54 - 2.49(m, 4H), 2.45 - 2.42(m, 1H), 2.39 - 2.33(m, 2H), 1.90 - 1.81(m, 1H), 1.71 - 1.63(m, 1H), 1.27(d, J = 6.2 Hz, 6H). MS: ESI m/z 640.1 [M+H]+

### Example 29. Preparation of N-(5-((5-chloro-4-((2-(1-methyl-1H-pyrazol-3-yl)phenyl)amino)pyrimidin-2-yl)amino)-2-((2-(dimethylamino)ethyl)(methyl)amino)-4-methoxyphenyl)acrylamide

In Step 1-1, 2,4,5-trichloropyrimidine is used, and except for Step 1-1, all experiments use the method of Example 1.

Yield : 28.4%, Light Yellow solid, 1H NMR(400 MHz, DMSO-d6) δ 11.34(s, 1H), 10.07(s, 1H), 8.57(d, J = 8.1 Hz, 1H), 8.39(s, 1H), 8.33(s, 1H), 8.10(s, 1H), 7.82(d, J = 2.3 Hz, 1H), 7.68(dd, J = 7.6, 1.9 Hz, 1H), 7.06 - 6.92(m, 3H), 6.76(d, J = 2.4 Hz, 1H), 6.35(dd, J = 16.9, 10.1 Hz, 1H), 6.13(dd, J = 17.0, 2.0 Hz, 1H), 5.68(dd, J = 10.1, 2.1 Hz, 1H), 3.91(s, 3H), 3.72(s, 3H), 2.84(t, J = 5.9 Hz, 2H), 2.69(s, 3H), 2.28(t, J = 5.7 Hz, 2H), 2.17(s, 6H). MS: ESI m/z 576.2586 [M+H]+

### Example 30. Preparation of N-(5-((4-((2-(1H-pyrazol-1-yl)phenyl)amino)-5-chloropyrimidin-2-yl)amino)-2-((2-(dimethylamino)ethyl)(methyl)amino)-4-methoxyphenyl)acrylamide

In Step 1-1, 2,4,5-trichloropyrimidine is used, and except for Step 1-1, all experiments use the method of Example 1.

Yield : 37.6%, Off-white solid, 1H NMR(400 MHz, DMSO-D6) δ 10.30(s, 1H), 10.07(s, 1H), 8.34(d, J = 14.0 Hz, 2H), 8.29(s, 1H), 8.24(d, J = 2.4 Hz, 1H), 8.06(s, 1H), 7.88(d, J = 1.8 Hz, 1H), 7.49(dd, J = 7.5, 2.0 Hz, 1H), 7.16 - 7.04(m, 2H), 6.96(s, 1H), 6.58 - 6.53(m, 1H), 6.35(dd, J = 16.9, 10.1 Hz, 1H), 6.15(dd, J = 16.9, 2.1 Hz, 1H), 5.74 - 5.66(m, 1H), 3.72(s, 3H), 2.83(t, J = 5.9 Hz, 2H), 2.68(s, 3H), 2.26(t, J = 5.9 Hz, 2H), 2.16(s, 6H). MS: ESI m/z 562.2453 [M+H]+

### Example 31. Preparation of N-(5-((5-chloro-4-((2-(1-methyl-1H-pyrazol-3-yl)phenyl)amino)pyrimidin-2-yl)amino)-4-methoxy-2-(4-methylpiperizin-1-yl)phenyl)acrylamide

In Step 1-1, 2,4,5-trichloropyrimidine is used, except for Step 1-1, all experiments use the method of Example 1 and in Step 3, 1-methylpiperizine is used.

Yield : 20.0%, Light Yellow solid, 1H NMR(400 MHz, DMSO-d6) δ 11.34(s, 1H), 10.07(s, 1H), 8.57(d, J = 8.1 Hz, 1H), 8.39(s, 1H), 8.33(s, 1H), 8.10(s, 1H), 7.82(d, J = 2.3 Hz, 1H), 7.68(dd, J = 7.6, 1.9 Hz, 1H), 7.06 - 6.92(m, 3H), 6.76(d, J = 2.4 Hz, 1H), 6.35(dd, J = 16.9, 10.1 Hz, 1H), 6.13(dd, J = 17.0, 2.0 Hz, 1H), 5.68(dd, J = 10.1, 2.1 Hz, 1H), 3.91(s, 3H), 3.72(s, 3H), 2.83 - 2.80(m, 4H), 2.42(m, 4H), 2.20(s, 3H). MS: ESI m/z 574.1 [M+H]+

### Example 32. Preparation of N-(5-((5-chloro-4-((2-(1-methyl-1H-pyrazol-3-yl)phenyl)amino)pyrimidin-2-yl)amino)-4-methoxy-2-morpholinophenyl)acrylamide

In Step 1-1, 2,4,5-trichloropyrimidine is used, except for Step 1-1, all experiments use the method of Example 1 and in Step 3, morpholine is used.

Yield : 23.2%, Off-white solid, 1H NMR(400 MHz, DMSO-d6) δ 11.34(s, 1H), 10.07(s, 1H), 8.57(d, J = 8.1 Hz, 1H), 8.39(s, 1H), 8.33(s, 1H), 8.10(s, 1H), 7.82(d, J = 2.3 Hz, 1H), 7.68(dd, J = 7.6, 1.9 Hz, 1H), 7.06 - 6.92(m, 3H), 6.76(d, J = 2.4 Hz, 1H), 6.35(dd, J = 16.9, 10.1 Hz, 1H), 6.13(dd, J = 17.0, 2.0 Hz, 1H), 5.68(dd, J = 10.1, 2.1 Hz, 1H), 3.91(s, 3H), 3.72(s, 3H), 3.71 - 3.68(m, 4H), 2.81 - 2.79(m, 4H). MS: ESI m/z 561.0 [M+H]+

### Example 33. Preparation of (S)-N-(5-((5-chloro-4-((2-(1-methyl-1H-pyrazol-3-yl)phenyl)amino)pyrimidin-2-yl)amino)-2-(3-(dimethylamino)pyrrolidin-1-yl)-4-methoxyphenyl)acrylamide

In Step 1-1, 2,4,5-trichloropyrimidine is used, except for Step 1-1, all experiments use the method of Example 1 and in Step 3, (S)-N,N-dimethylpyrrolidin-3-amine is used.

Yield : 19.0%, Off-white solid, 1H NMR(400 MHz, DMSO-d6) δ 11.34(s, 1H), 10.07(s, 1H), 8.57(d, J = 8.1 Hz, 1H), 8.39(s, 1H), 8.33(s, 1H), 8.10(s, 1H), 7.82(d, J = 2.3 Hz, 1H), 7.68(dd, J = 7.6, 1.9 Hz, 1H), 7.06 - 6.92(m, 3H), 6.76(d, J = 2.4 Hz, 1H), 6.35(dd, J = 16.9, 10.1 Hz, 1H), 6.13(dd, J = 17.0, 2.0 Hz, 1H), 5.68(dd, J = 10.1, 2.1 Hz, 1H), 3.91(s, 3H), 3.72(s, 3H), 3.37 - 3.30(m, 1H), 3.17 - 3.13(m, 3H), 2.64 - 2.57(m, 1H), 2.11(s, 6H), 2.05 - 1.99(m, 1H), 1.70 - 1.61(m, 1H). MS: ESI m/z 588.0 [M+H]+

### Example 34. Preparation of (R)-N-(5-((5-chloro-4-((2-(1-methyl-1H-pyrazol-3-yl)phenyl)amino)pyrimidin-2-yl)amino)-2-(3-(dimethylamino)pyrrolidin-1-yl)-4-methoxyphenyl)acrylamide

In Step 1-1, 2,4,5-trichloropyrimidine is used, except for Step 1-1, all experiments use the method of Example 1 and in Step 3, (R)-N,N-dimethylpyrrolidin-3-amine is used.

Yield : 19.0%, Off-white solid, 1H NMR(400 MHz, DMSO-d6) δ 11.34(s, 1H), 10.07(s, 1H), 8.57(d, J = 8.1 Hz, 1H), 8.39(s, 1H), 8.33(s, 1H), 8.10(s, 1H), 7.82(d, J = 2.3 Hz, 1H), 7.68(dd, J = 7.6, 1.9 Hz, 1H), 7.06 - 6.92(m, 3H), 6.76(d, J = 2.4 Hz, 1H), 6.35(dd, J = 16.9, 10.1 Hz, 1H), 6.13(dd, J = 17.0, 2.0 Hz, 1H), 5.68(dd, J = 10.1, 2.1 Hz, 1H), 3.91(s, 3H), 3.72(s, 3H), 3.37 - 3.30(m, 1H), 3.17 - 3.13(m, 3H), 2.64 - 2.57(m, 1H), 2.11(s, 6H), 2.05 - 1.99(m, 1H), 1.70 - 1.61(m, 1H). MS: ESI m/z 588.0 [M+H]+

### Example 35. Preparation of (R)-N-(5-((5-chloro-4-((2-(1-methyl-1H-pyrazol-3-yl)phenyl)amino)pyrimidin-2-yl)amino)-4-methoxy-2-(methyl(1-methylpyrrolidin-3-yl)amino)phenyl)acrylamide

In Step 1-1, 2,4,5-trichloropyrimidine is used, except for Step 1-1, all experiments use the method of Example 1 and in Step 3, (R)-N,1-dimethylpyrrolidin-3-amine is used.

Yield : 20.3%, Off-white solid, 1H NMR(400 MHz, DMSO-d6) δ 11.34(s, 1H), 10.07(s, 1H), 8.57(d, J = 8.1 Hz, 1H), 8.39(s, 1H), 8.33(s, 1H), 8.10(s, 1H), 7.82(d, J = 2.3 Hz, 1H), 7.68(dd, J = 7.6, 1.9 Hz, 1H), 7.06 - 6.92(m, 3H), 6.76(d, J = 2.4 Hz, 1H), 6.35(dd, J = 16.9, 10.1 Hz, 1H), 6.13(dd, J = 17.0, 2.0 Hz, 1H), 5.68(dd, J = 10.1, 2.1 Hz, 1H), 3.91(s, 3H), 3.72(s, 3H), 3.58 - 3.51(m, 1H), 2.54 - 2.49(m, 4H), 2.45 - 2.42(m, 1H), 2.39 - 2.33(m, 2H), 1.90 - 1.81(m, 1H), 1.71 - 1.63(m, 1H). MS: ESI m/z 588.0 [M+H]+

### Example 36. Preparation of (S)-N-(5-((5-chloro-4-((2-(1-methyl-1H-pyrazol-3-yl)phenyl)amino)pyrimidin-2-yl)amino)-4-methoxy-2-(methyl(1-methylpyrrolidin-3-yl)amino)phenyl)acrylamide

In Step 1-1, 2,4,5-trichloropyrimidine is used, except for Step 1-1, all experiments use the method of Example 1 and in Step 3, (S)-N,1-dimethylpyrrolidin-3-amine is used.

Yield : 19.7%, Off-white solid, 1H NMR(400 MHz, DMSO-d6) δ 11.34(s, 1H), 10.07(s, 1H), 8.57(d, J = 8.1 Hz, 1H), 8.39(s, 1H), 8.33(s, 1H), 8.10(s, 1H), 7.82(d, J = 2.3 Hz, 1H), 7.68(dd, J = 7.6, 1.9 Hz, 1H), 7.06 - 6.92(m, 3H), 6.76(d, J = 2.4 Hz, 1H), 6.35(dd, J = 16.9, 10.1 Hz, 1H), 6.13(dd, J = 17.0, 2.0 Hz, 1H), 5.68(dd, J = 10.1, 2.1 Hz, 1H), 3.91(s, 3H), 3.72(s, 3H), 3.58 - 3.51(m, 1H), 2.54 - 2.49(m, 4H), 2.45 - 2.42(m, 1H), 2.39 - 2.33(m, 2H), 1.90 - 1.81(m, 1H), 1.71 - 1.63(m, 1H). MS: ESI m/z 588.0 [M+H]+

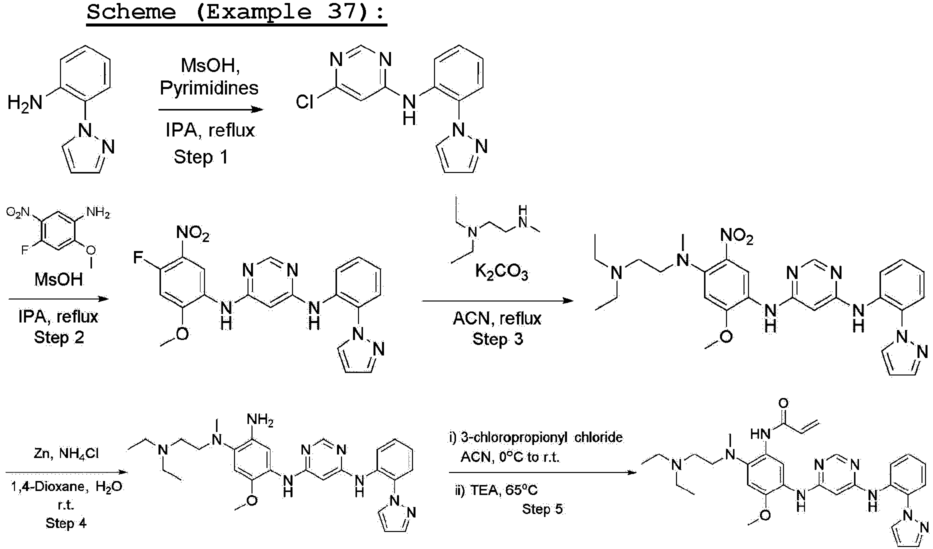

### Step 1: Synthesis of N-(2-(1H-pyrazol-1-yl)phenyl)-6-chloropyrimidin-4-amine

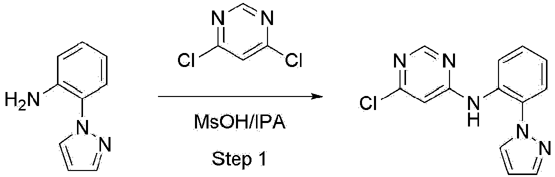

A pyrimidine derivative(1.0 equiv.) is dissolved in isopropyl alcohol, and an aniline derivative(1.0 equiv.) and methanesulfonyl acid(1.3 equiv.) are added thereto at room temperature, followed by stirring overnight at 80°C. After completion of the reaction, the solvent is removed by evaporation under reduced pressure, and extraction is performed using water and ethyl acetate. The organic layer is evaporated under reduced pressure and subjected to column chromatography to obtain the target compound.

### Step 2: Synthesis of N4-(2-(1H-pyrazol-1-yl)phenyl)-N6-(4-fluoro-2-methoxy-5-nitrophenyl)pyrimidin-4,6-diamine

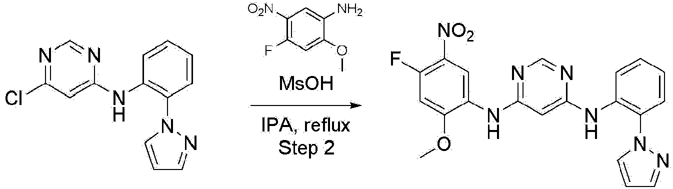

A pyrimidine derivative (1.0 equiv.) is dissolved in isopropyl alcohol, and an aniline derivative (1.0 equiv.) and methanesulfonyl acid (1.3 equiv.) are added thereto at room temperature, followed by stirring overnight at 80°C. After completion of the reaction, the solvent is removed by evaporation under reduced pressure, and extraction is performed using water and ethyl acetate. The organic layer is evaporated under reduced pressure and subjected to column chromatography to obtain the target compound.

### Step 3: Synthesis of N4-(2-(1H-pyrazol-1-yl)phenyl)-N6-(4-((2-(diethylamino)ethyl) (methyl)amino)-2-methoxy-5-nitrophenyl)pyrimidin-4,6-diamine

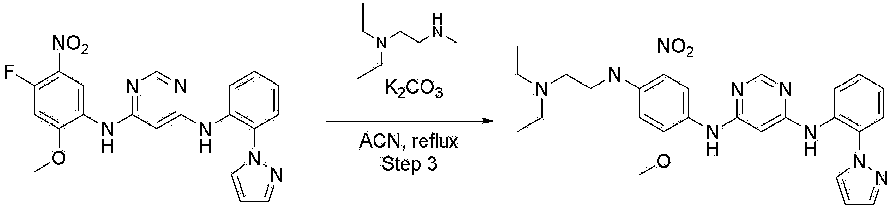

N4-(2-(1H-pyrazol-1-yl)phenyl)-N6-(4-fluoro-2-methoxy-5-nitrophenyl)pyrimidin-4,6-diamine (1.0 equiv.) is dissolved in acetonitrile, and potassium carbonate (3.0 equiv.) and amine chain (1.2 equiv.) are added thereto at room temperature followed by refluxing and stirring overnight. After completion of the reaction, the temperature was lowered to room temperature and filtration is performed. The filtrate is evaporated under reduced pressure and subjected to column chromatography to obtain the target compound. (methylene chloride: methanol=10: 1)

### Step 4: Synthesis of N4-(6-((2-(1H-pyrazol-1-yl)phenyl)amino)pyrimidin-4-yl)-N1-(2-(diethylamino)ethyl)-5-methoxy-N1-methylbenzene-1,2,4-triamine

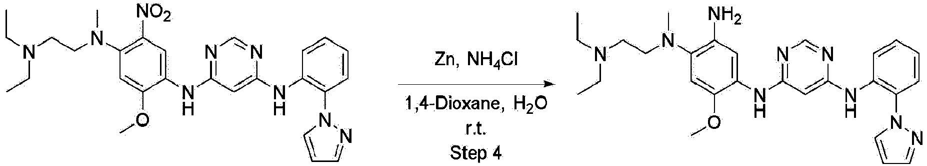

N4-(2-(1H-pyrazol-1-yl)phenyl)-N6-(4-((2-(diethylamino)ethyl)(methyl)amino)-2-methoxy-5-nitrophenyl)pyrimidin-4,6-diamine (1.0 equiv.) is dissolved in 1,4-dioxane and zinc(10.0 equiv.) and ammonium chloride (10.0 equiv.) are added thereto at room temperature, followed by stirring overnight at room temperature. After completion of the reaction, the temperature is lowered to room temperature, and the filtrate is extracted using water and ethyl acetate after filtering with celite. The organic layer is collected, dried, and then evaporated under reduced pressure to obtain a compound. It is used in the next reaction without separation.

### Step 5: Synthesis of N-(5-((6-((2-(1H-pyrazol-1-yl)phenyl)amino)pyrimidin-4-yl)amino)-2-((2-(diethylamino)ethyl)(methyl)amino)-4-methoxyphenyl)acrylamide

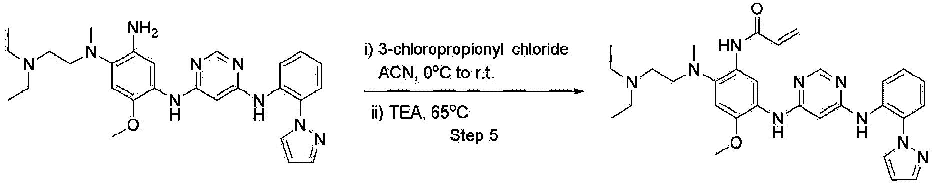

N4-(6-((2-(1H-pyrazol-1-yl)phenyl)amino)pyrimidin-4-yl)-N1-(2-(diethylamino)ethyl)-5-methoxy-N1-methylbenzene-1,2,4-triamine (1.0 equiv.) is dissolved in acetonitrile and 3-chloropropionyl chloride (1.2 equiv.) is added thereto at 0±5°C, followed by stirring for 15 minutes at the same temperature. After completion of the reaction, triethylamine (4.0 equiv.) is added at the same temperature. The reactor temperature is raised and stirred overnight at 65°C. After completion of the reaction, the solvent is removed by evaporation under reduced pressure and extraction is performed using water and methylene chloride. The organic layer is evaporated under reduced pressure and subjected to column chromatography to obtain the target compound. (methylene chloride: methanol=10: 1)

### Example 37. Preparation of N-(5-((6-((2-(1H-pyrazol-1-yl)phenyl)amino)pyrimidin-4-yl)amino)-2-((2-(diethylamino)ethyl)(methyl)amino)-4-methoxyphenyl)acrylamide

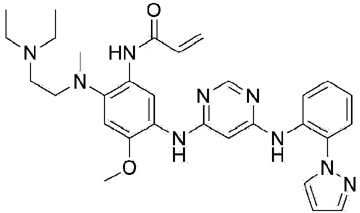

Yield : 35.0%, White solid, 1H NMR (400 MHz, DMSO-d6) δ 9.64 (s, 1H), 8.96 (s, 1H), 8.36 (s, 1H), 8.27 (s, 1H), 8.10 (dd, J = 2.5, 0.6 Hz, 1H), 8.03 (d, J = 0.9 Hz, 1H), 7.75 (dd, J = 1.8, 0.6 Hz, 1H), 7.73 (dd, J = 8.2, 1.4 Hz, 1H), 7.50 (dd, J = 8.0, 1.5 Hz, 1H), 7.32 (td, J = 7.7, 1.6 Hz, 1H), 7.18 (td, J = 7.6, 1.4 Hz, 1H), 6.88 (s, 1H), 6.48 (dd, J = 2.5, 1.8 Hz, 1H), 6.41 (dd, J = 16.9, 10.1 Hz, 1H), 6.19 (dd, J = 17.0, 2.1 Hz, 1H), 5.92 (d, J = 1.0 Hz, 1H), 5.70 (dd, J = 10.0, 2.0 Hz, 1H), 3.73 (s, 3H), 3.33 - 3.25 (m, 2H), 2.78 (t, J = 6.1 Hz, 2H), 2.64 (s, 3H), 2.49 (m, 2H), 2.44 - 2.38 (m, 2H), 0.90 (t, J = 7.1 Hz, 6H). MS: ESI m/z 556.3151 [M+H]+

### Example 38. Preparation of N-(5-((6-((2-(1H-pyrazol-1-yl)phenyl)amino)pyrimidin-4-yl)amino)-4-methoxy-2-(tetrahydro-1H-furo[3,4-c]pyrrol-5(3H)-yl)phenyl)acrylamide

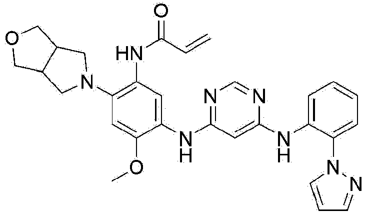

Synthesis was performed using hexahydro-1H-furo[3,4-c]pyrrole in Step 3.

Yield : 31.0%, White solid, 1H NMR (400 MHz, DMSO-d6) δ 9.10 (s, 1H), 8.95 (s, 1H), 8.25 (s, 1H), 8.10 (d, J = 2.5 Hz, 1H), 8.02 (s, 1H), 7.82 - 7.70 (m, 3H), 7.51 (dd, J = 7.9, 1.5 Hz, 1H), 7.34 (t, J = 7.8 Hz, 1H), 7.19 (t, J = 7.6 Hz, 1H), 6.63 (s, 1H), 6.54 - 6.40 (m, 2H), 6.22 - 6.11 (m, 1H), 5.90 (s, 1H), 5.73 - 5.62 (m, 1H), 3.79 (t, J = 7.3 Hz, 2H), 3.74 (s, 3H), 3.56 - 3.41 (m, 2H), 3.14 - 3.02 (m, 2H), 2.82 (m, 4H). MS: ESI m/z 539.2550 [M+H]+

### Example 39. Preparation of N-(5-((6-((2-(1H-pyrazol-1-yl)phenyl)amino)pyrimidin-4-yl)amino)-2-(3-oxa-8-azabicyclo[3.2.1]octan-8-yl)-4-methoxyphenyl)acrylamide

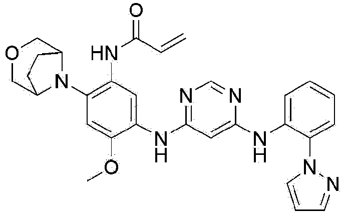

Synthesis was performed using 3-oxa-8-azabicyclo[3.2.1]octane in Step 3.

Yield : 30.0%, White solid, 1H NMR (400 MHz, DMSO-d6) δ 9.02 (s, 1H), 8.95 (s, 1H), 8.23 (s, 1H), 8.10 (d, J = 2.4 Hz, 1H), 8.02 (s, 1H), 7.82 - 7.68 (m, 3H), 7.51 (dd, J = 8.0, 1.5 Hz, 1H), 7.33 (t, J = 7.8 Hz, 1H), 7.19 (t, J = 7.6 Hz, 1H), 6.55 (dd, J = 17.0, 10.2 Hz, 1H), 6.48 (q, J = 2.0, 1.5 Hz, 2H), 6.17 (dd, J = 17.1, 1.9 Hz, 1H), 5.88 (s, 1H), 5.67 (dd, J = 10.5, 1.7 Hz, 1H), 3.81 (d, J = 10.1 Hz, 2H), 3.73 (s, 3H), 3.58 (s, 2H), 3.50 (d, J = 10.0 Hz, 2H), 1.84 (s, 4H). MS: ESI m/z 539.2533 [M+H]+

**[Table 1]**

| Compounds of Examples 1 to 39 | | | |
|---|---|---|---|
| | Structure | ESI-MS [M+H]⁺ | Name |
| 1 | 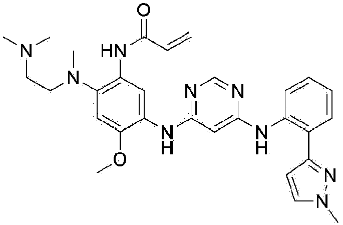 | 542.1 | N-(2-((2-(dimethylamino) ethyl) (methyl) amino)-4-methoxy-5-((6-((2-(1-methyl-1H-pyrazol-3-yl)phenyl)amino)pyrimidin-4-yl)amino) phenyl) acrylamide |
| 2 | 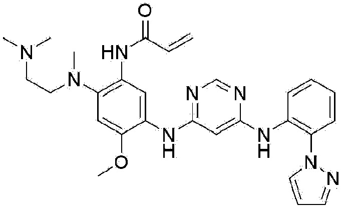 | 528.3 | N-(5-((6-((2-(1H-pyrazol-1-yl)phenyl)amino)pyrimidin-4-yl)amino)-2-((2-(dimethylamino) ethyl) (methyl) amino)-4-methoxyphenyl)acrylamide |
| 3 | 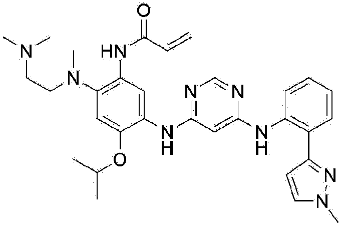 | 570.1 | N-(2-((2-(dimethylamino) ethyl) (methyl) amino)-4-isopropoxy-5-((6-((2-(1-methyl-1H-pyrazol-3-yl)phenyl)amino)pyrimidin-4-yl)amino)phenyl)acrylamide |
| 4 | 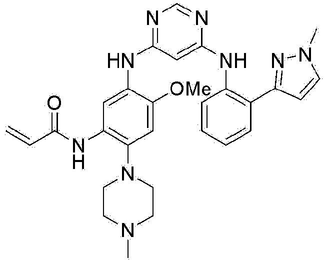 | 540.0 | N-(4-methoxy-5-((6-((2-(1-methyl-1H-pyrazol-3-yl)phenyl)amino)pyrimidin-4-yl)amino)-2-(4-methylpiperizin-1-yl)phenyl)acrylamide |
| 5 | 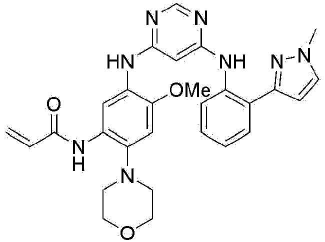 | 527.0 | N-(4-methoxy-5-((6-((2-(1-methyl-1H-pyrazol-3-yl)phenyl)amino)pyrimidin-4-yl)amino)-2-morpholinophenyl)acrylamide |
| 6 | 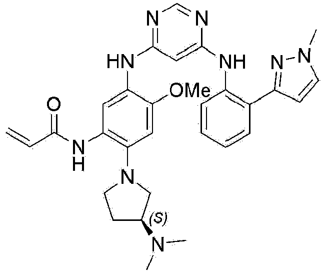 | 534.1 | (S)-N-(2-(3-(dimethylamino)pyrrolidin-1-yl)-4-methoxy-5-((6-((2-(1-methyl-1H-pyrazol-3-yl)phenyl)amino)pyrimidin-4-yl)amino) phenyl) acrylamide |
| 7 | 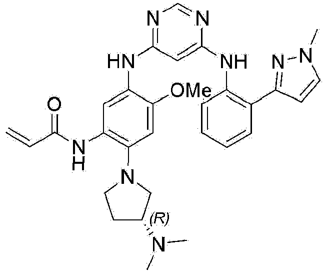 | 534.1 | (R) -N- (2- (3-(dimethylamino)pyrrolidin-1-yl)-4-methoxy-5-((6-((2-(1-methyl-1H-pyrazol-3-yl)phenyl)amino)pyrimidin-4-yl)amino) phenyl) acrylamide |
| 8 | 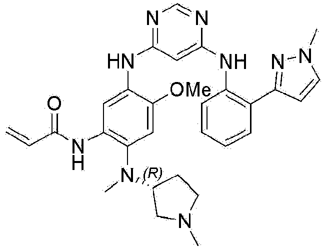 | 534.1 | (R)-N-(4-methoxy-2-(methyl(1-methylpyrrolidin-3-yl)amino)-5-((6-((2-(1-methyl-1H-pyrazol-3-yl)phenyl)amino)pyrimidin-4-yl)amino) phenyl) acrylamide |
| 9 | 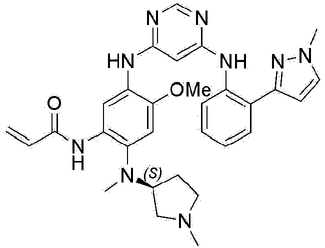 | 534.1 | (S)-N-(4-methoxy-2-(methyl(1-methylpyrrolidin-3-yl)amino)-5-((6-((2-(1-methyl-1H-pyrazol-3-yl)phenyl)amino)pyrimidin-4-yl)amino) phenyl) acrylamide |
| 10 | 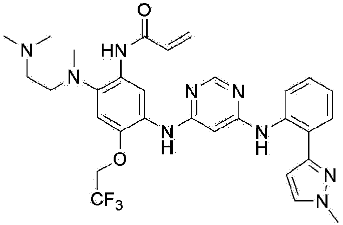 | 610.1 | N-(2-((2-(diamethylamino) ethyl) (methy )amino)-5-((6-((2-(1-methyl-1H-pyrazol-3-yl)phenyl)amino)pyrimidin-4-yl)amino)-4-(2,2,2-trifluoroethoxy)phenyl)acryla mide |
| 11 | 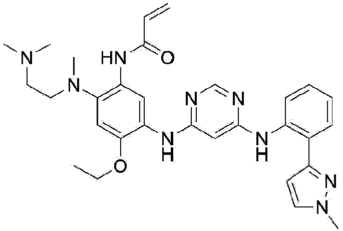 | 556.1 | N-(2-((2-(dimethylamino) ethyl) (methyl) amino)-4-ethoxy-5-((6-((2-(1-methyl-1H-pyrazol-3-yl)phenyl)amino)pyrimidin-4-yl)amino)phenyl)acetamide |
| 12 | 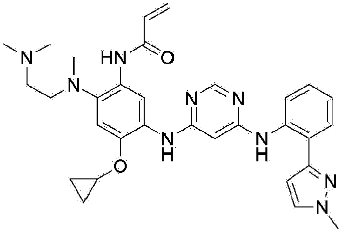 | 568.1 | N-(4-cyclopropoxy-2-((2-(dimethylamino) ethyl) (methyl) amino)-5-((6-((2-(1-methyl-1H-pyrazol-3-yl)phenyl)amino)pyrimidin-4-yl)amino) phenyl) acrylamide |
| 13 | 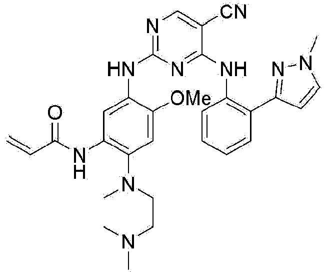 | 567.1 | N-(5-((5-cyano-4-((2-(1-methyl-1H-pyrazol-3-yl)phenyl)amino)pyrimidin-2-yl)amino)-2-((2-(dimethylamino) ethyl) (methyl) amino)-4-methoxyphenyl)acrylamide |
| 14 | 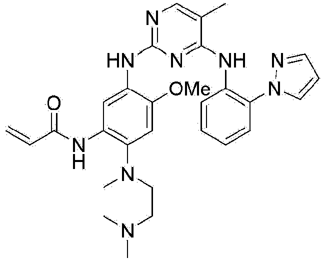 | 542.1 | N-(5-((4-((2-(1H-pyrazol-1-yl)phenyl)amino)-5-methylpyrimidin-2-yl)amino)-2-((2-(dimethylamino) ethyl) (methyl) amino)-4-methoxyphenyl)acrylamide |
| 15 | 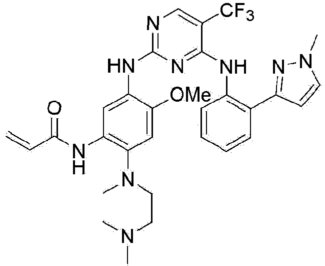 | 610.0 | N-(2-((2-(dimethylamino) ethyl) (methyl) amino)-4-methoxy-5-((4-((2-(1-methyl-1H-pyrazol-3-yl)phenyl)amino)-5-(trifluoromethyl)pyrimidin-2-yl)amino) phenyl) acrylamide |
| 16 | 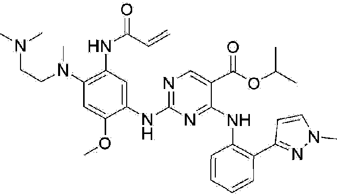 | 628.2 | Isopropyl 2-((5-acrylamido-4-((2-(dimethylamino) ethyl) (methyl) amino)-2-methoxyphenyl)amino)-4-((2-(1-methyl-1H-pyrazol-3-yl)phenyl)amino)pyrimidin-5-carboxylate |
| 17 | 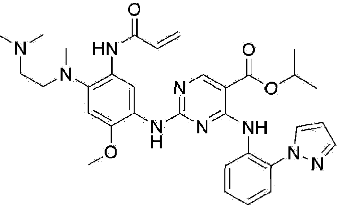 | 614.1 | Isopropyl 4-((2-(1H-pyrazol-1-yl)phenyl)amino)-2-((5-acrylamido-4-((2-(dimethylamino) ethyl) (methyl) amino)-2-methoxyphenyl)amino)pyrimidin -5-carboxylate |
| 18 | 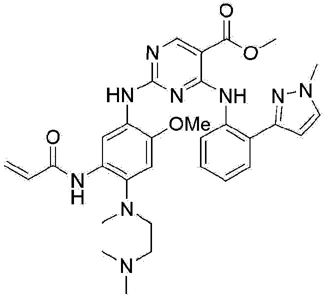 | 600.1 | Methyl 2-((5-acrylamido-4-((2-(dimethylamino) ethyl) (methyl) amino)-2-methoxyphenyl)amino)-4-((2-(1-methyl-1H-pyrazol-3-yl)phenyl)amino)pyrimidin-5-carboxylate |
| 19 | 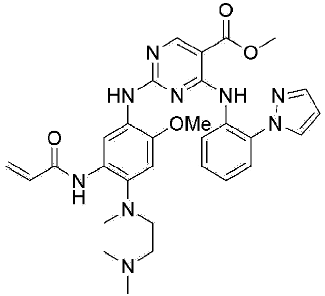 | 586.1 | Methyl 4-((2- (1H-pyrazol-1-yl)phenyl)amino)-2-((5-acrylamido-4-((2-(dimethylamino) ethyl) (methyl) amino)-2-methoxyphenyl)amino)pyrimidin -5-carboxylate |
| 20 | 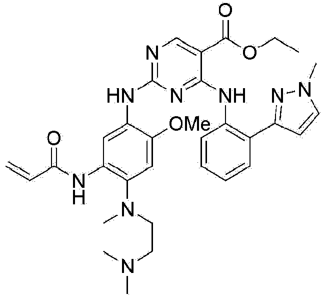 | 614.1 | Ethyl 2-((5-acrylamido-4-((2-(dimethylamino) ethyl) (methyl) amino)-2-methoxyphenyl)amino)-4-((2-(1-methyl-1H-pyrazol-3-yl)phenyl)amino)pyrimidin-5-carboxylate |
| 21 | 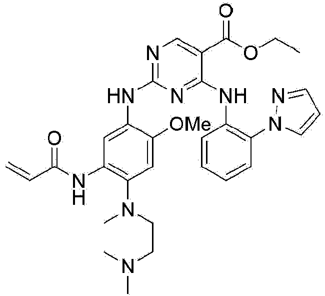 | 600.1 | Ethyl 4-((2-(1H-pyrazol-1-yl)phenyl)amino)-2-((5-acrylamido-4-((2-(dimethylamino) ethyl) (methyl) amino)-2-methoxyphenyl)amino)pyrimidin -5-carboxylate |
| 22 | 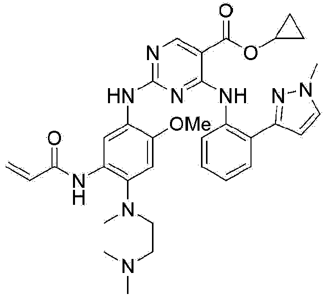 | 626.1 | Cyclopropyl 2-((5-acrylamido-4-((2-(dimethylamino) ethyl) (methyl) amino)-2-methoxyphenyl)amino)-4-((2-(1-methyl-1H-pyrazol-3-yl)phenyl)amino)pyrimidin-5-carboxylate |
| 23 | 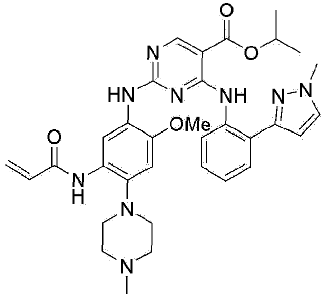 | 626.1 | Isopropyl 2-((5-acrylamido-2-methoxy-4-(4-methylpiperizin-1-yl)phenyl)amino)-4-((2-(1-methyl-1H-pyrazol-3-yl)phenyl)amino)pyrimidin-5-carboxylate |
| 24 | 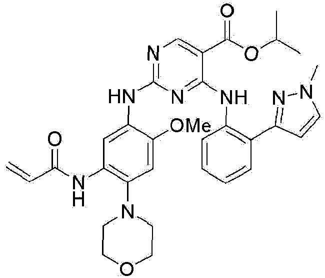 | 613.1 | Isopropyl 2-((5-acrylamido-2-methoxy-4-morpholinophenyl)amino)-4-((2-(1-methyl-1H-pyrazol-3-yl)phenyl)amino)pyrimidin-5-carboxylate |
| 25 | 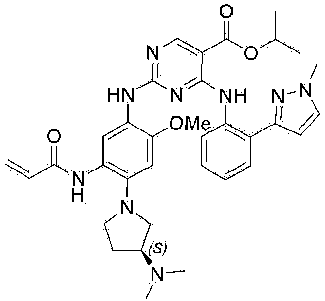 | 640.1 | Isopropyl(S)-2-((5-acrylamido-4-(3-(dimethylamino)pyrrolidin-1-yl)-2-methoxyphenyl)amino)-4-((2-(1-methyl-1H-pyrazol-3-yl)phenyl)amino)pyrimidin-5-carboxylate |
| 26 | 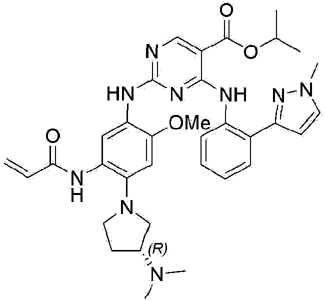 | 640.1 | Isopropyl(R)-2-((5-acrylamido-4-(3-(dimethylamino)pyrrolidin-1-yl)-2-methoxyphenyl)amino)-4-((2-(1-methyl-1H-pyrazol-3-yl)phenyl)amino)pyrimidin-5-carboxylate |
| 27 | 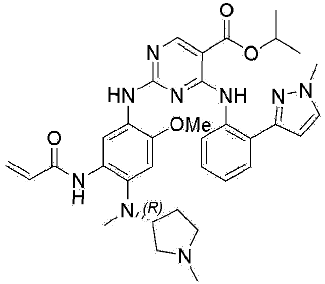 | 640.1 | Isopropyl(R)-2-((5-acrylamido-2-methoxy-4-(methyl (1-methylpyrrolidin-3-yl)amino)phenyl)amino)-4-((2-(1-methyl-1H-pyrazol-3-yl)phenyl)amino)pyrimidin-5-carboxylate |
| 28 | 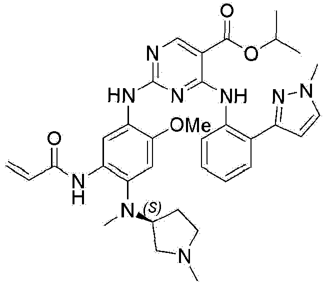 | 640.1 | Isopropyl(S)-2-((5-acrylamido-2-methoxy-4-(methyl(1-methylpyrrolidin-3-yl)amino)phenyl)amino)-4-((2-(1-methyl-1H-pyrazol-3-yl)phenyl)amino)pyrimidin-5-carboxylate |
| 29 | 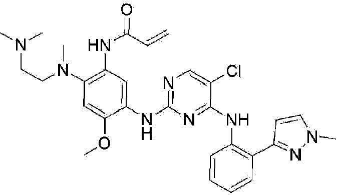 | 576.3 | N-(5-((5-chloro-4-((2-(1-methyl-1H-pyrazol-3-yl)phenyl)amino)pyrimidin-2-yl)amino)-2-((2-(dimethylamino) ethyl) (methyl) amino)-4-methoxyphenyl)acrylamide |
| 30 | 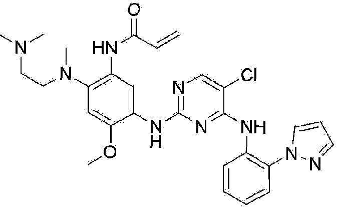 | 562.2 | N-(5-((4-((2-(1H-pyrazol-1-yl)phenyl)amino)-5-chloropyrimidin-2-yl)amino)-2-((2-(dimethylamino)ethyl)(methyl) amino)-4-methoxyphenyl)acrylamide |
| 31 | 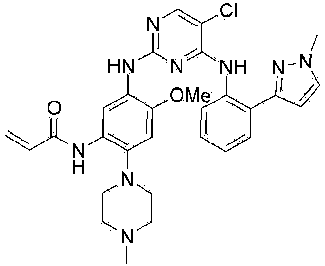 | 574.1 | N-(5-((5-chloro-4-((2-(1-methyl-1H-pyrazol-3-yl)phenyl)amino)pyrimidin-2-yl)amino)-4-methoxy-2-(4-methylpiperizin-1-yl)phenyl)acrylamide |
| 32 | 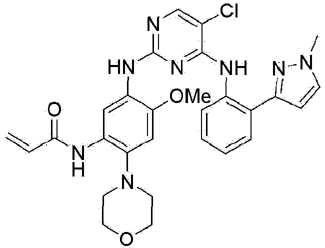 | 561.0 | N-(5-((5-chloro-4-((2-(1-methyl-1H-pyrazol-3-yl)phenyl)amino)pyrimidin-2-yl)amino)-4-methoxy-2-morpholinophenyl)acrylamide |
| 33 | 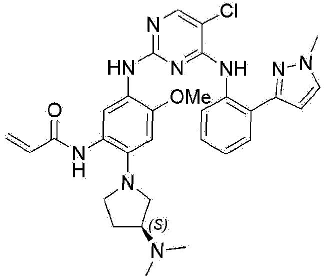 | 588.0 | (S)-N-(5-((5-chloro-4-((2-(1-methyl-1H-pyrazol-3-yl)phenyl)amino)pyrimidin-2-yl)amino)-2-(3-(dimethylamino)pyrrolidin-1-yl)-4-methoxyphenyl)acrylamide |
| 34 | 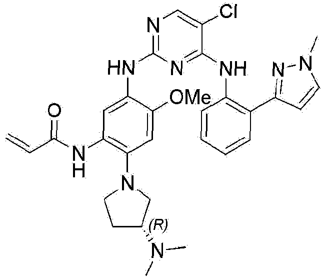 | 588.0 | (R)-N-(5-((5-chloro-4-((2-(1-methyl-1H-pyrazol-3-yl)phenyl)amino)pyrimidin-2-yl)amino)-2-(3-(dimethylamino)pyrrolidin-1-yl)-4-methoxyphenyl)acrylamide |
| 35 | 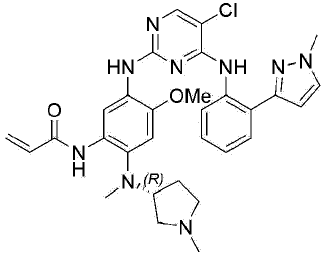 | 588.0 | (R)-N-(5-((5-chloro-4-((2-(1-methyl-1H-pyrazol-3-yl)phenyl)amino)pyrimidin-2-yl)amino)-4-methoxy-2-(methyl (1-methylpyrrolidin-3-yl)amino) phenyl) acrylamide |
| 36 | 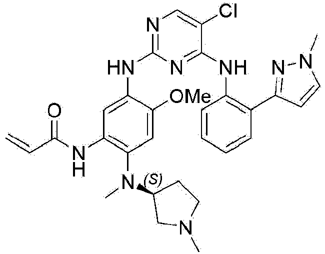 | 588.0 | (S)-N-(5-((5-chloro-4-((2-(1-methyl-1H-pyrazol-3-yl)phenyl)amino)pyrimidin-2-yl)amino)-4-methoxy-2-(methyl (1-methylpyrrolidin-3-yl)amino) phenyl) acrylamide |
| 37 | 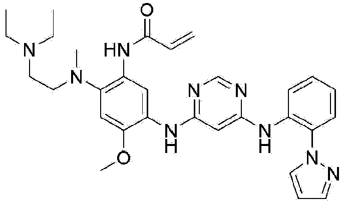 | 556.3151 | N-(5-((6-((2-(1H-pyrazol-1-yl)phenyl)amino)pyrimidin-4-yl)amino)-2-((2-(diethylamino) ethyl) (methyl) a mino)-4-methoxyphenyl)acrylamide |
| 38 | 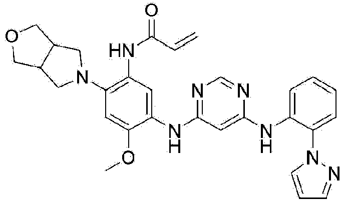 | 539.2550 | N-(5-((6-((2-(1H-pyrazol-1-yl)phenyl)amino)pyrimidin-4-yl)amino)-4-methoxy-2-(tetrahydro-1H-furo[3,4-c]pyrrol-5(3H)-yl)phenyl)acrylamide |
| 39 | 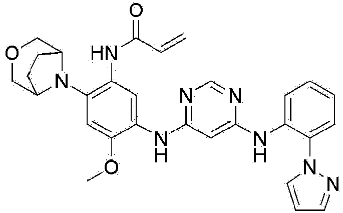 | 539.2533 | N-(5-((6-((2-(1H-pyrazol-1-yl)phenyl)amino)pyrimidin-4-yl)amino)-2-(3-oxa-8-azabicyclo[3.2.1]octan-8-yl)-4-methoxyphenyl)acrylamide |

### <Test Example> Measurement of cancer cell growth inhibitory effect

Since there is no commercially available lung cancer cell line with EGFR insertion mutation, a method of inserting 3 to 4 amino acids at each site using site direct mutagenesis in a wild type EGFR expression vector was attempted. In the present invention, first, a vector with three amino acids of NPG added to the D770_N771 site with the highest frequency was used, and Ba/F3 cell line was used for expression of these genes.

The Ba/F3 cell line is a Murine pro B cell line that shows cell growth only when IL-3 is added. When the oncogenic mutant EGFR is expressed, dependence on the mutant EGFR for cell growth and apoptosis occurs without IL-3. Therefore, substances that show effective inhibition of mutant EGFR suppress cell growth and induce apoptosis, so the anticancer effect of cells was analyzed through MTT assay.

1×10⁴ cells of the previously constructed stable cells were put into a 96 well plate and incubated overnight, and then the compound of Example was dose dependently treated. After 72 hours, MTT reagent was added, and after 3 hours stop buffer (10% SDS) was added. After 24 hours of incubation, the results were analyzed by reading at 595 nm. IC₅₀ values were calculated at the concentration at which each compound inhibited cell growth by 50%, and the results are shown as A, B, and C in Table 1 below(wherein, A means IC₅₀ of less than 25nM, B means IC₅₀ of 25 to 50nM, and C means IC₅₀ of 50 to 500nM) . As a control drug, Mobocertinib was used.

**[Table 2]**

| Measurement of cancer cell growth inhibitory effect(IC₅₀) | |
|---|---|
| Example | EXON 20 Insertion (NPG) Ba/F3 Cell (IC₅₀) |
| 1 | A |
| 2 | A |
| 3 | B |
| 4 | B |
| 5 | B |
| 6 | B |
| 7 | B |
| 8 | B |
| 9 | B |
| 10 | A |
| 11 | B |
| 12 | B |
| 13 | B |
| 14 | B |
| 15 | B |
| 16 | A |
| 17 | A |
| 18 | B |
| 19 | B |
| 20 | B |
| 21 | B |
| 22 | B |
| 23 | B |
| 24 | B |
| 25 | B |
| 26 | B |
| 27 | B |
| 28 | B |
| 29 | A |
| 30 | A |
| 31 | B |
| 32 | B |
| 33 | B |
| 34 | B |
| 35 | B |
| 36 | B |
| 37 | B |
| 38 | B |
| 39 | B |
| Mobocertinib | C |

As shown in Table 2, the compounds prepared by Examples of the present invention showed excellent activity against epidermal growth factor receptor exon 20 insertion mutation-expressing lung cancer cell lines. The compounds of the Examples showed equal or superior activity to the control drug, Mobocertinib. Therefore, the present invention proposes a novel pyrimidine derivative that can treat diseases associated with epidermal growth factor receptor exon 20 insertion mutation gene or protein expression or cancer showing epidermal growth factor receptor exon 20 insertion mutation.

## Claims

1. A compound represented by Formula I below, a solvate, a stereoisomer or a pharmaceutically acceptable salt thereof: 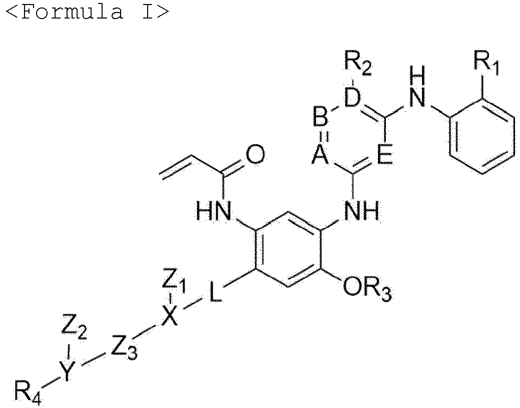 wherein,
A, B, and E are each independently N or CH,
D is N or C,
X is CH, N or O,
Y is CH, N or O,
L is a single bond or NR₅,
R₅ is H or C1 to C4 alkyl,
Z₁ and Z₂ are each independently C1 to C4 alkyl, or each independently contain carbon and are linked to each other to form a 5- to 8-membered ring with X and Y,
Z₃ is (CH₂)ₙ, or contains carbon and forms a 5- to 8-membered ring with Z₁, Z₂, X and Y,
n is an integer from 1 to 3,
R₁ is H, pyrazole, or pyrazole substituted with C1 to C4 alkyl,
R₂ is H, halogen, C1 to C4 alkyl, C1 to C4 alkyl ester, CN, or C1 to C4 alkyl substituted with halogen (However, if D is N, R₂ does not exist),
R₃ is H, C1 to C5 linear or branched chain alkyl, C1 to C5 alkyl substituted with halogen, or C3 to C5 cycloalkyl, and
R₄ does not exist, or is H, C1 to C4 alkyl, C1 to C4 monoalkyl, or C1 to C4 dialkylamine.

2. The compound, solvate, stereoisomer, pharmaceutically acceptable salt thereof according to claim 1, wherein the Z₁ and Z₂ each independently contain carbon and are connected to each other to form a 5- to 8-membered monocyclic, fused bicyclic, or bridged bicyclic ring with X and Y.

3. The compound, solvate, stereoisomer, pharmaceutically acceptable salt thereof according to claim 1, wherein A and D in Formula I above are N.

4. The compound, solvate, stereoisomer, pharmaceutically acceptable salt thereof according to claim 1, wherein A and E in Formula I above are N.

5. The compound, solvate, stereoisomer, pharmaceutically acceptable salt thereof according to claim 1, wherein in Formula I above, L is a single bond, and X is N or O.

6. The compound, solvate, stereoisomer, pharmaceutically acceptable salt thereof according to claim 1, wherein in Formula I above, L is NR₅, and X is CH,
wherein the Z₁ and Z₂ each independently contain carbon and are connected to each other to form a 5- to 8-membered monocyclic, fused bicyclic, or bridged bicyclic ring with Z₃, X and Y.

7. The compound, solvate, stereoisomer, pharmaceutically acceptable salt thereof according to claim 1, which is selected from the group consisting of the following compounds:
N-(2-((2-(dimethylamino)ethyl)(methyl)amino)-4-methoxy-5-((6-((2-(1-methyl-1H-pyrazol-3-yl)phenyl)amino)pyrimidin-4-yl)amino)phenyl)acrylamide;
N-(5-((6-((2-(1H-pyrazol-1-yl)phenyl)amino)pyrimidin-4-yl)amino)-2-((2-(dimethylamino)ethyl)(methyl)amino)-4-methoxyphenyl)acrylamide;
N-(2-((2-(dimethylamino)ethyl)(methyl)amino)-4-isopropoxy-5-((6-((2-(1-methyl-1H-pyrazol-3-yl)phenyl)amino)pyrimidin-4-yl)amino)phenyl)acrylamide;
N-(4-methoxy-5-((6-((2-(1-methyl-1H-pyrazol-3-yl)phenyl)amino)pyrimidin-4-yl)amino)-2-(4-methylpiperizin-1-yl)phenyl)acrylamide;
N-(4-methoxy-5-((6-((2-(1-methyl-1H-pyrazol-3-yl)phenyl)amino)pyrimidin-4-yl)amino)-2-morpholinophenyl)acrylamide;
(S)-N-(2-(3-(dimethylamino)pyrrolidin-1-yl)-4-methoxy-5-((6-((2-(1-methyl-1H-pyrazol-3-yl)phenyl)amino)pyrimidin-4-yl)amino)phenyl)acrylamide;
(R)-N-(2-(3-(dimethylamino)pyrrolidin-1-yl)-4-methoxy-5-((6-((2-(1-methyl-1H-pyrazol-3-yl)phenyl)amino)pyrimidin-4-yl)amino)phenyl)acrylamide;
(R)-N-(4-methoxy-2-(methyl(1-methylpyrrolidin-3-yl)amino)-5-((6-((2-(1-methyl-1H-pyrazol-3-yl)phenyl)amino)pyrimidin-4-yl)amino)phenyl)acrylamide;
(S)-N-(4-methoxy-2-(methyl(1-methylpyrrolidin-3-yl)amino)-5-((6-((2-(1-methyl-1H-pyrazol-3-yl)phenyl)amino)pyrimidin-4-yl)amino)phenyl)acrylamide;
N-(2-((2-(diamethylamino)ethyl)(methyl)amino)-5-((6-((2-(1-methyl-1H-pyrazol-3-yl)phenyl)amino)pyrimidin-4-yl)amino)-4-(2,2,2-trifluoroethoxy)phenyl)acrylamide;
N-(2-((2-(dimethylamino)ethyl)(methyl)amino)-4-ethoxy-5-((6-((2-(1-methyl-1H-pyrazol-3-yl)phenyl)amino)pyrimidin-4-yl)amino)phenyl)acetamide;
N-(4-cyclopropoxy-2-((2-(dimethylamino)ethyl)(methyl)amino)-5-((6-((2-(1-methyl-1H-pyrazol-3-yl)phenyl)amino)pyrimidin-4-yl)amino)phenyl)acrylamide;
N-(5-((5-cyano-4-((2-(1-methyl-1H-pyrazol-3-yl)phenyl)amino)pyrimidin-2-yl)amino)-2-((2-(dimethylamino)ethyl)(methyl)amino)-4-methoxyphenyl)acrylamide;
N-(5-((4-((2-(1H-pyrazol-1-yl)phenyl)amino)-5-methylpyrimidin-2-yl)amino)-2-((2-(dimethylamino)ethyl)(methyl)amino)-4-methoxyphenyl)acrylamide;
N-(2-((2-(dimethylamino)ethyl)(methyl)amino)-4-methoxy-5-((4-((2-(1-methyl-1H-pyrazol-3-yl)phenyl)amino)-5-(trifluoromethyl)pyrimidin-2-yl)amino)phenyl)acrylamide;
Isopropyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate;
Isopropyl 4-((2-(1H-pyrazol-1-yl)phenyl)amino)-2-((5-acrylamido-4-((2-(dimethylamino)ethyl)(methyl)amino)-2-methoxyphenyl)amino)pyrimidin-5-carboxylate;
Methyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate;
Methyl 4-((2-(1H-pyrazol-1-yl)phenyl)amino)-2-((5-acrylamido-4-((2-(dimethylamino)ethyl)(methyl)amino)-2-methoxyphenyl)amino)pyrimidin-5-carboxylate;
Ethyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate;
Ethyl 4-((2-(1H-pyrazol-1-yl)phenyl)amino)-2-((5-acrylamido-4-((2-(dimethylamino)ethyl)(methyl)amino)-2-methoxyphenyl)amino)pyrimidin-5-carboxylate;
Cyclopropyl 2-((5-acrylamido-4-((2-(dimethylamino)ethyl)(methyl)amino)-2-methoxyphenyl)amino)-4-((2-(1-methyl-1H-pyrazol-3-yl)phenyl)amino)pyrimidin-5-carboxylate;
Isopropyl 2-((5-acrylamido-2-methoxy-4-(4-methylpiperizin-1-yl)phenyl)amino)-4-((2-(1-methyl-1H-pyrazol-3-yl)phenyl)amino)pyrimidin-5-carboxylate;
Isopropyl 2-((5-acrylamido-2-methoxy-4-morpholinophenyl)amino)-4-((2-(1-methyl-1H-pyrazol-3-yl)phenyl)amino)pyrimidin-5-carboxylate;
Isopropyl(S)-2-((5-acrylamido-4-(3-(dimethylamino)pyrrolidin-1-yl)-2-methoxyphenyl)amino)-4-((2-(1-methyl-1H-pyrazol-3-yl)phenyl)amino)pyrimidin-5-carboxylate;
Isopropyl(R)-2-((5-acrylamido-4-(3-(dimethylamino)pyrrolidin-1-yl)-2-methoxyphenyl)amino)-4-((2-(1-methyl-1H-pyrazol-3-yl)phenyl)amino)pyrimidin-5-carboxylate;
Isopropyl(R)-2-((5-acrylamido-2-methoxy-4-(methyl(1-methylpyrrolidin-3-yl)amino)phenyl)amino)-4-((2-(1-methyl-1H-pyrazol-3-yl)phenyl)amino)pyrimidin-5-carboxylate;
Isopropyl(S)-2-((5-acrylamido-2-methoxy-4-(methyl(1-methylpyrrolidin-3-yl)amino)phenyl)amino)-4-((2-(1-methyl-1H-pyrazol-3-yl)phenyl)amino)pyrimidin-5-carboxylate;
N-(5-((5-chloro-4-((2-(1-methyl-1H-pyrazol-3-yl)phenyl)amino)pyrimidin-2-yl)amino)-2-((2-(dimethylamino)ethyl)(methyl)amino)-4-methoxyphenyl)acrylamide;
N-(5-((4-((2-(1H-pyrazol-1-yl)phenyl)amino)-5-chloropyrimidin-2-yl)amino)-2-((2-(dimethylamino)ethyl)(methyl)amino)-4-methoxyphenyl)acrylamide;
N-(5-((5-chloro-4-((2-(1-methyl-1H-pyrazol-3-yl)phenyl)amino)pyrimidin-2-yl)amino)-4-methoxy-2-(4-methylpiperizin-1-yl)phenyl)acrylamide;
N-(5-((5-chloro-4-((2-(1-methyl-1H-pyrazol-3-yl)phenyl)amino)pyrimidin-2-yl)amino)-4-methoxy-2-morpholinophenyl)acrylamide;
(S)-N-(5-((5-chloro-4-((2-(1-methyl-1H-pyrazol-3-yl)phenyl)amino)pyrimidin-2-yl)amino)-2-(3-(dimethylamino)pyrrolidin-1-yl)-4-methoxyphenyl)acrylamide;
(R)-N-(5-((5-chloro-4-((2-(1-methyl-1H-pyrazol-3-yl)phenyl)amino)pyrimidin-2-yl)amino)-2-(3-(dimethylamino)pyrrolidin-1-yl)-4-methoxyphenyl)acrylamide;
(R)-N-(5-((5-chloro-4-((2-(1-methyl-1H-pyrazol-3-yl)phenyl)amino)pyrimidin-2-yl)amino)-4-methoxy-2-(methyl(1-methylpyrrolidin-3-yl)amino)phenyl)acrylamide;
(S)-N-(5-((5-chloro-4-((2-(1-methyl-1H-pyrazol-3-yl)phenyl)amino)pyrimidin-2-yl)amino)-4-methoxy-2-(methyl(1-methylpyrrolidin-3-yl)amino)phenyl)acrylamide;
N-(5-((6-((2-(1H-pyrazol-1-yl)phenyl)amino)pyrimidin-4-yl)amino)-2-((2-(diethylamino)ethyl)(methyl)amino)-4-methoxyphenyl)acrylamide;
N-(5-((6-((2-(1H-pyrazol-1-yl)phenyl)amino)pyrimidin-4-yl)amino)-4-methoxy-2-(tetrahydro-1H-furo[3,4-c]pyrrol-5(3H)-yl)phenyl)acrylamide; and
N-(5-((6-((2-(1H-pyrazol-1-yl)phenyl)amino)pyrimidin-4-yl)amino)-2-(3-oxa-8-azabicyclo[3.2.1]octan-8-yl)-4-methoxyphenyl)acrylamide.

8. A pharmaceutical composition for preventing or treating a disease over-expressing tyrosine kinase domain mutant EGFR, comprising the compound, solvate, stereoisomer or pharmaceutically acceptable salt thereof according to any one of claims 1 to 7.

9. The pharmaceutical composition according to claim 8, wherein the pharmaceutical composition further comprises a pharmaceutically acceptable carrier.

10. The pharmaceutical composition according to claim 8, wherein the mutant EGFR is EGFR exon 20 insertion.

11. The pharmaceutical composition according to claim 8, wherein the disease over-expressing mutant EGFR is a cancer.

12. The pharmaceutical composition according to claim 11, wherein the cancer is at least one selected from the group consisting of liver cancer, hepatocellular carcinoma, gastrointestinal cancer, stomach cancer, meningioma associated with neurofibromatosis, pancreatic cancer, leukemia, myeloproliferative disease, myelodysplastic disease, dermatofibrosarcoma, breast cancer, lung cancer, thyroid cancer, colorectal cancer, prostate cancer, ovarian cancer, brain tumor, head and neck cancer and glioblastoma.

13. The pharmaceutical composition according to claim 11, wherein the cancer is non-small cell lung cancer.

14. A method for preventing or treating a disease over-expressing tyrosine kinase domain mutant EGFR, comprising a step of administering the compound, solvate, stereoisomer or pharmaceutically acceptable salt thereof according to any one of claims 1 to 7 to a subject.

15. The compound, solvate, stereoisomer or pharmaceutically acceptable salt thereof according to any one of claims 1 to 7, for use in the prevention or treatment of a disease over-expressing tyrosine kinase domain mutant EGFR.

16. A use of the compound, solvate, stereoisomer or pharmaceutically acceptable salt thereof according to any one of claims 1 to 7 for the prevention or treatment of a disease over-expressing tyrosine kinase domain mutant EGFR.

17. A use of the compound, solvate, stereoisomer or pharmaceutically acceptable salt thereof according to any one of claims 1 to 7 for the prevention or treatment of a disease over-expressing tyrosine kinase domain mutant EGFR.
